# EUROPÄISCHE PATENTSCHRIFT

(11) **EP 2 594 626 B1**
(45) Veröffentlichungstag und Bekanntmachung des Hinweises auf die Patenterteilung: **25.06.2014**
(21) Anmeldenummer: 11189681.7
(22) Anmeldetag: 18.11.2011
(51) Int. Cl.: C11B 9/00, A61Q 13/00, A61K 8/33, C07C 43/315, C07C 47/198, C07C 47/277

(54) **Verwendung von Oxyacetaldehyden als Maiglöckchen-Riechstoffe**
Use of oxyacetaldehyde as lily of the valley fragrance
Utilisation d'oxyacétaldéhydes comme parfums sentant le muguet

(43) Veröffentlichungstag der Anmeldung: 22.05.2013
(73) Patentinhaber: Symrise AG, 37603 Holzminden (DE)
(72) Erfinder: Singer, Emilie, 37603 Holzminden (DE)
(74) Vertreter: Fabry, Bernd

(56) Entgegenhaltungen:
- EP-A1- 1 857 436
- EP-A2- 0 335 290
- EP-A2- 1 170 278
- WO-A1-92/00267
- WO-A1-2004/076393
- WO-A1-2010/017986
- WO-A2-2008/092678

## Beschreibung

Die vorliegende Erfindung betrifft primär die Verwendung bestimmter Verbindungen aus der Gruppe der Oxyacetaldehyde als Riechstoffe mit einer Maiglöckchen-Note.

Dabei wird erfindungsgemäß eine Verbindung der Formel (I) oder eine Mischung enthaltend eine oder mehrere Verbindungen der Formel (I) oder eine Mischung bestehend aus zwei oder mehreren Verbindungen der Formel (I) 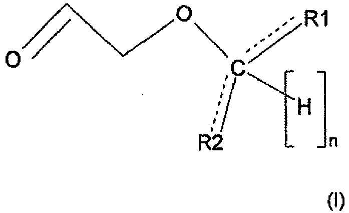 verwendet als
- Riechstoff mit einer Maiglöckchen-Note
und/oder
- Mittel zum Erhöhen der Substantivität und/oder der Retention einer Riechstoffzubereitung
und/oder
- Fixateur.

In der Formel (I) gilt:
n ist 0 oder 1, wobei
   (i) wenn n = 1
      die beiden Paare aus einer durchgezogenen und einer gestrichelten Linie in der Formel (I) jeweils Einfachbindungen darstellen
      und
      a) R1 und R2 jeweils eine unverzweigte gesättigte Alkylgruppe darstellen, wobei die Gesamtanzahl der Kohlenstoffatome in den Gruppen R1 und R2 4 bis 9 beträgt
         oder
      b) R1 und R2 gemeinsam mit dem zwischen ihnen angeordneten Kohlenstoffatom einen Cyclohexylring 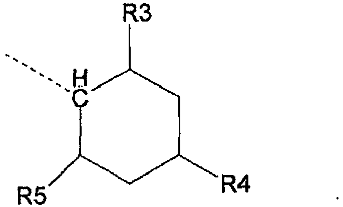 bilden,
         wobei die gestrichelte Linie die Bindung darstellt, die den Cyclohexylring mit dem in Formel (I) benachbarten Sauerstoffatom verbindet, und
         wobei R3, R4 und R5 unabhängig voneinander ausgewählt sind aus der Gruppe bestehend aus Wasserstoff, Methyl, Ethyl, Isopropyl und tert-Butyl und wobei die Gesamtanzahl der Kohlenstoffatome des Cyclohexylrings und der Gruppen R3, R4 und R5 6 bis 10 beträgt
         oder
      c) R1 Wasserstoff ist und R2
         c1) eine verzweigte gesättigte Alkylgruppe mit 4 bis 9 Kohlenstoffatomen ist
            oder
         c2) ein Cyclohexylring 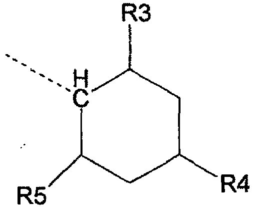 ist, wobei die gestrichelte Linie die Bindung darstellt, die den Cyclohexylring mit dem in Formel (I) benachbarten Kohlenstoffatom verbindet, und
            wobei R3, R4 und R5 unabhängig voneinander ausgewählt sind aus der Gruppe bestehend aus Wasserstoff, Methyl, Ethyl und Isopropyl und die Gesamtanzahl der Kohlenstoffatome des Cyclohexylrings und der Gruppen R3, R4 und R5 6 bis 9 beträgt oder
         c3) ein Phenylring 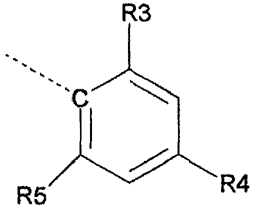 ist, wobei die gestrichelte Linie die Bindung darstellt, die den Phenylring mit dem in Formel (I) benachbarten Kohlenstoffatom verbindet, und
            wobei R3, R4 und R5 unabhängig voneinander ausgewählt sind aus der Gruppe bestehend aus Wasserstoff, Methyl, Ethyl und Isopropyl und die Gesamtanzahl der Kohlenstoffatome des Phenylrings und der Gruppen R3, R4 und R5 6 bis 9 beträgt
      und
   (ii) wenn n = 0
      die beiden Paare aus einer durchgezogenen und einer gestrichelten Linie in der Formel (I) jeweils aromatische Bindungen darstellen
   und
   (d) R1 und R2 gemeinsam mit dem zwischen ihnen angeordneten Kohlenstoffatom einen Phenylring 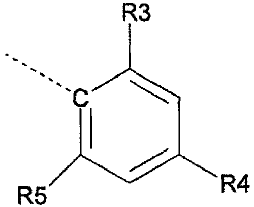 bilden,
      wobei die gestrichelte Linie die Bindung darstellt, die den Phenylring mit dem in Formel (I) benachbarten Sauerstoffatom verbindet, und
      wobei R3, R4 und R5 unabhängig voneinander ausgewählt sind aus der Gruppe bestehend aus Wasserstoff, Methyl, Ethyl, Isopropyl und tert-Butyl und die Gesamtanzahl der Kohlenstoffatome des Phenylrings und der Gruppen R3, R4 und R5 6 bis 10 beträgt.

Daneben betrifft die Erfindung Mischungen enthaltend eine oder mehrere Verbindungen der Formel (I) wie oben definiert sowie bestimmte Verbindungen der Formel (I) wie oben definiert.

Verbindungen mit blumigem Geruch sind eine unverzichtbare Komponente in der Riechstoffindustrie sowie bei der Herstellung von Kosmetika, Körperpflegemitteln sowie Wasch- und Reinigungsmitteln. Eine besonders wertvolle Klasse dieser blumigen Riechstoffe sind Verbindungen mit einer Maiglöckchen-Geruchsnote.

3-(4-Tertbutylphenyl)-2-methylpropanal (Lilial®, Formel (A)), 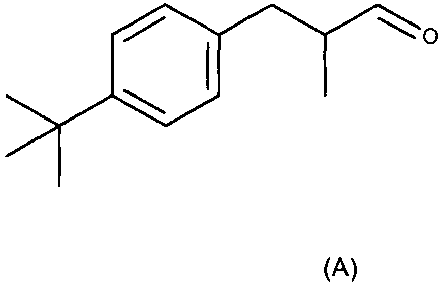 ein synthetischer Riechstoff, war über lange Zeit einer der bedeutendsten industriell eingesetzten Riechstoffe für blumige Duftkompositionen, insbesondere mit Maiglöckchen-Geruchsnote, und wurde in der Kosmetik- und Seifenindustrie in großen Mengen verwendet. Dann zeigten jedoch Resultate von Tierversuchen, dass 3-(4-Tertbutylphenyl)-2-methylpropanal reprotoxisch sein könnte. Außerdem wurde festgestellt, dass diese Verbindung möglicherweise ein Allergen ist und Kontakt-Dermatitis bei empfindlichen Personen verursachen könnte.

Das Auftreten eines Geruchs mit einer Maiglöckchennote ist nicht an das oben beschriebene strukturelle Grundgerüst gebunden. So sind auch Verbindungen bekannt, die trotz struktureller Ähnlichkeit zu 3-(4-Tertbutylphenyl)-2-methylpropanal (A) keine Maiglöckchen-Geruchsnote aufweisen, während andererseits Riechstoffe bekannt sind, die eine Maiglöckchen-Geruchsnote ähnlich der von 3-(4-Tertbutylphenyl)-2-methylpropanal (A) aufweisen, obwohl sie sich strukturell signifikant von 3-(4-Tertbutylphenyl)-2-methylpropanal (A) unterscheiden.

WO 2004/0769 offenbart die Verwendung von 4-Cyclohexyl-2-butanol als Riechstoff und zwar insbesondere als Maiglöckchen-Riechstoff.

EP 1 857 436 offenbart einen Precursor für Duftstoffe vom Aldehyd-Typ oder Keton-Typ.

Die Suche nach Riechstoffen mit einer bestimmten Geruchsnote wird daher im Allgemeinen durch folgende Sachverhalte erschwert:
- die Mechanismen der Geruchswahrnehmung sind nicht ausreichend bekannt;
- die Zusammenhänge zwischen der speziellen Geruchswahrnehmung einerseits und der chemischen Struktur des zugehörigen Riechstoffs andererseits sind nicht hinreichend erforscht;
- häufig bewirken bereits geringfügige Änderungen am strukturellen Aufbau eines bekannten Riechstoffs starke Änderungen der sensorischen Eigenschaften und beeinträchtigen die Verträglichkeit für den menschlichen Organismus.

Der Erfolg der Suche nach geeigneten Riechstoffen hängt deshalb stark von der Intuition des Suchenden ab.

Trotz einer Vielzahl bereits vorhandener Riechstoffe besteht in der Parfümindustrie ein genereller Bedarf an neuen Riechstoffen, welche über ihre primären, nämlich geruchlichen, Eigenschaften hinaus zusätzliche positive Sekundäreigenschaften besitzen, wie z.B. eine höhere Stabilität unter bestimmten Anwendungsbedingungen, eine höhere Substantivität und/oder Diffusivität oder ein besseres Haftungsvermögen, oder welche durch Synergieeffekte mit anderen Riechstoffen zu besseren sensorischen Profilen führen.

Riechstoffe, die sich durch die oben erwähnten positiven Sekundäreigenschaften auszeichnen, ermöglichen eine erhöhte Effektivität bei der Herstellung von Riechstoffzubereitungen und parfümierten Produkten. So können z.B. durch den Einsatz von Riechstoffen mit einem besseren sensorischen Profil, einer höheren Substantivität und/oder einem besseren Haftungsvermögen die Anzahl und die Einsatzmengen von Riechstoffen in entsprechenden Formulierungen optimiert und/oder minimiert werden, was zu einer nachhaltigen Ressourcenschonung bei der Herstellung parfümierter Produkte führt.

Daher besteht in der Parfümindustrie insbesondere ein Bedarf an weiteren Riechstoffen mit verbesserten sensorischen Profilen und/oder Sekundäreigenschaften.

Primäre Aufgabe der vorliegenden Erfindung war es, zur Verwendung als Riechstoff mit einer Maiglöckchen-Geruchsnote geeignete Verbindungen anzugeben, deren geruchliche Eigenschaften vergleichbar mit 3-(4-Tertbutylphenyl)-2-methylpropanal (Lilial®) sind, das heißt, bei Verwendung der entsprechenden Verbindung als Riechstoff soll ein Geruchseindruck vermittelt werden, der der Komplexität des natürlichen Geruchs der Maiglöckchenblüte weitgehend entspricht. Darüber hinaus sollte dieser Riechstoff vorzugsweise hinsichtlich der Summe seiner Sekundäreigenschaften bzw. zumindest einiger seiner Sekundäreigenschaften den aus dem Stand der Technik bekannten Maiglöckchen-Riechstoffen überlegen sein.

Ein Riechstoff ist im Rahmen des vorliegenden Textes jede Substanz, die dazu geeignet ist, zum Hervorrufen eines geruchlichen Eindrucks verwendet zu werden, d.h. zum Vermitteln eines geruchlichen Eindrucks oder zum Verändern (Modifizieren oder Verstärken) der geruchlichen Wahrnehmung einer anderen Substanz. Dabei soll diese Substanz, damit sie für parfümistische Zwecke einsetzbar ist, vorzugsweise keine unerwünschten Nebenwirkungen, z.B. die Gesundheit oder die Umwelt schädigende Wirkungen, oder den bestimmungsgemäßen Gebrauch eines Produkts, das diesen Riechstoff enthält, beeinträchtigende Wirkungen haben.

Die gestellte Aufgabe wird erfindungsgemäß gelöst durch die Verwendung einer Verbindung der Formel (I) 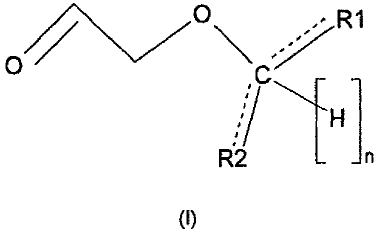 worin gilt:
n ist 0 oder 1 (nachstehend Fälle (i) und (ii)).

Im Fall (i) gilt:
Die beiden Paare aus einer durchgezogenen und einer gestrichelten Linie in der Formel (I) stellen jeweils Einfachbindungen dar, d.h. die Verbindung der Formel (I) hat im Fall (i) die folgende Struktur (I'): 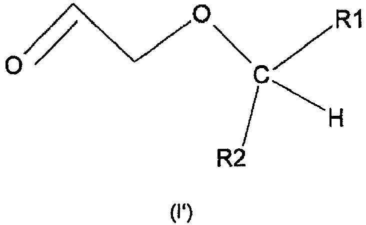
worin
a) R1 und R2 jeweils eine unverzweigte gesättigte Alkylgruppe darstellen, wobei die Gesamtanzahl der Kohlenstoffatome in den Gruppen R¹ und R² 4 bis 9 beträgt oder
b) R1 und R2 gemeinsam mit dem zwischen ihnen angeordneten Kohlenstoffatom einen Cyclohexylring 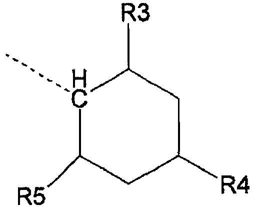 bilden,
   wobei die gestrichelte Linie die Bindung darstellt, die den Cyclohexylring mit dem in Formel (I') benachbarten Sauerstoffatom verbindet, und
   wobei R3, R4 und R5 unabhängig voneinander ausgewählt sind aus der Gruppe bestehend aus Wasserstoff, Methyl, Ethyl, Isopropyl und tert-Butyl und wobei die Gesamtanzahl der Kohlenstoffatome des Cyclohexylrings und der Gruppen R3, R4 und R5 6 bis 10 beträgt,
   d.h. die Verbindung der Formel (I) hat im Fall (i)b) die folgende Struktur (I'b): 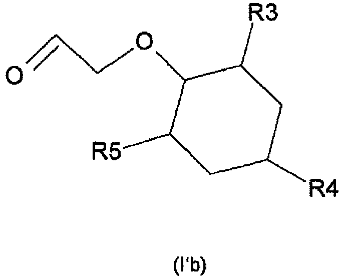 oder
c) R1 Wasserstoff ist und R2
   c1) eine verzweigte gesättigte Alkylgruppe mit 4 bis 9 Kohlenstoffatomen ist, d.h. die Verbindung der Formel (I) hat im Fall (i)c1) die folgende Struktur (I'c1): 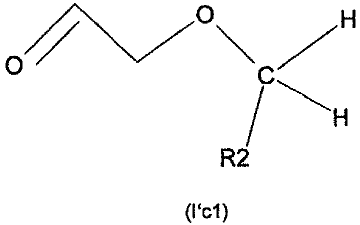 oder
   c2) ein Cyclohexylring 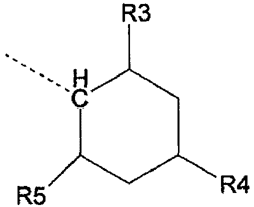 ist, wobei die gestrichelte Linie die Bindung darstellt, die den Cyclohexylring mit dem in Formel (I') benachbarten Kohlenstoffatom verbindet, und
      wobei R3, R4 und R5 unabhängig voneinander ausgewählt sind aus der Gruppe bestehend aus Wasserstoff, Methyl, Ethyl und Isopropyl und die Gesamtanzahl der Kohlenstoffatome des Cyclohexylrings und der Gruppen R3, R4 und R5 6 bis 9 beträgt,
      d.h. die Verbindung der Formel (I) hat im Fall (i)c2) die folgende Struktur (I'c2): 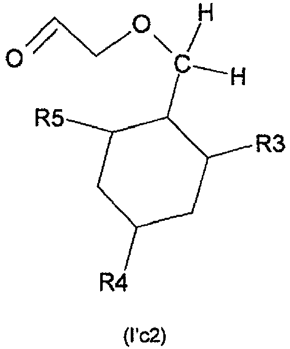 oder
   c3) ein Phenylring 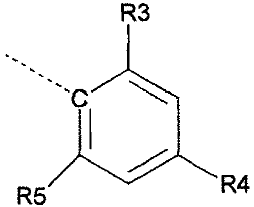 ist, wobei die gestrichelte Linie die Bindung darstellt, die den Phenylring mit dem in Formel (I') benachbarten Kohlenstoffatom verbindet, und
      wobei R3, R4 und R5 unabhängig voneinander ausgewählt sind aus der Gruppe bestehend aus Wasserstoff, Methyl, Ethyl und Isopropyl und die Gesamtanzahl der Kohlenstoffatome des Phenylrings und der Gruppen R3, R4 und R5 6 bis 9 beträgt,
      d.h. die Verbindung der Formel (I) hat im Fall (i)c3) die folgende Struktur (I'c3):

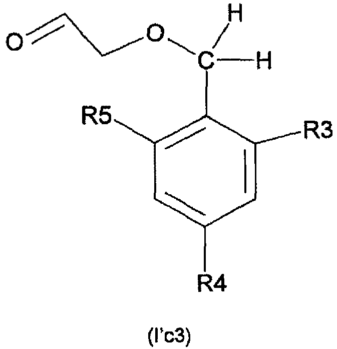

Im Fall (ii) gilt:
Die beiden Paare aus einer durchgezogenen und einer gestrichelten Linie in der Formel (I) stellen jeweils aromatische Bindungen dar, und
   (d) R1 und R2 bilden gemeinsam mit dem zwischen ihnen angeordneten Kohlenstoffatom einen Phenylring 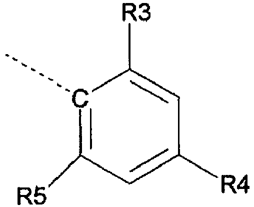 wobei die gestrichelte Linie die Bindung darstellt, die den Phenylring mit dem in Formel (I) benachbarten Sauerstoffatom verbindet, und
      wobei R3, R4 und R5 unabhängig voneinander ausgewählt sind aus der Gruppe bestehend aus Wasserstoff, Methyl, Ethyl, Isopropyl und tert-Butyl und die Gesamtanzahl der Kohlenstoffatome des Phenylrings und der Gruppen R3, R4 und R5 6 bis 10 beträgt,
d.h. die Verbindung der Formel (I) hat im Fall (II) die folgende Struktur (I"):

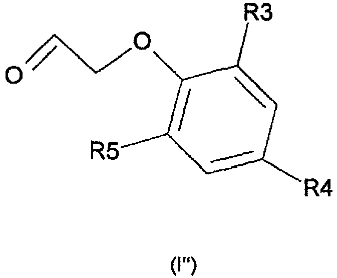

Die Verbindungen der Formel (I) können ihrer Struktur nach als Oxyacetaldehyde aufgefasst werden.

Weitere Aspekte der Erfindung ergeben sich aus der nachfolgenden Beschreibung, den Ausführungsbeispielen und den beigefügten Patentansprüchen.

Überraschenderweise hat sich gezeigt, dass die Verbindungen der Formel (I) wie oben definiert einen Geruchseindruck vermitteln, der der Komplexität des natürlichen Geruchs der Maiglöckchenblüte sehr nahe kommt. Das heißt, der von der Verbindung der Formel (I) vermittelte Geruchseindruck zeichnet sich insbesondere hinsichtlich der Maiglöckchennote durch eine herausragende Natürlichkeit und Komplexität aus. Ein derartig komplexer, dem natürlichen Geruch der Maiglöckchenblüte weitgehend entsprechender Geruchseindruck ist mit den aus dem Stand der Technik bekannten Riechstoffen mit Maiglöckchen-Geruchsnote bisher nicht erreicht worden.

Dass das Geruchsprofil der erfindungsgemäß einzusetzenden Verbindungen der Formel (I) dem Geruchsprofil von 3-(4-Tertbutylphenyl)-2-methylpropanal sehr ähnlich ist, war nicht zu erwarten, da sich die Verbindungen der Formel (I) strukturell signifikant von 3-(4-Tertbutylphenyl)-2-methylpropanal (obige Formel (A)) unterscheiden.

Die erfindungsgemäß einzusetzenden Verbindungen der Formel (I) zeichnen sich durch ein hohes Aufziehvermögen (Eigenhaftung auf einem Substrat) und eine hohe Substantivität (Fähigkeit, aus einer, meist wässrigen, Phase heraus auf ein Substrat aufzuziehen bzw. auch nach einem Wasch- oder Spülvorgang auf einem Substrat zu verbleiben) aus. Dieser Effekt zeigt sich insbesondere auf Substraten wie Haut, Haar und textilen Fasern (z.B. Wolle, Baumwolle, Leinen, synthetische Fasern).

Eine weitere wichtige anwendungstechnische Anforderung an Riechstoffe und Riechstoffzubereitungen, insbesondere für tensidhaltige Produkte, ist deren Substantivität gegenüber dem bzw. Retention am Substrat, insbesondere Haaren oder textilen Fasern. Die Begriffe "Substantivität" und "Retention" werden z.B. in EP 1 201 738 A1 ausführlich erläutert, vergleiche dort die Abschnitte [0004]-[0005]. Gesucht werden generell Riechstoffe mit hoher Substantivität und/oder Retention.

Diesen Erkenntnissen entsprechend betrifft ein weiterer Aspekt der Erfindung insbesondere die Verwendung der Verbindung der Formel (I) als Mittel zum Erhöhen der Substantivität und/oder Retention einer Riechstoffzubereitung (insbesondere gegenüber bzw. auf Haaren bzw. textilen Fasern), bevorzugt einer Riechstoffzubereitung mit Maiglöckchen-Geruchsnote, vorzugsweise einer erfindungsgemäßen Riechstoffzubereitung wie weiter unten beschrieben.

Neben ihrem hohen Aufziehvermögen zeichnen sich die erfindungsgemäß zu verwendenden Verbindungen der Formel (I) durch ihre fixierenden Eigenschaften aus, d.h. sie sind Fixateure. Als Fixateur erhöhen die erfindungsgemäß zu verwendenden Verbindungen der Formel (I) die Haftfestigkeit anderer Riechstoffe, sei es durch deren Dampfdruckerniedrigung oder geruchliche Verstärkung (z.B. Absenkung des Schwellenwertes). Die Erfindung betrifft daher auch - wie bereits erwähnt - die Verwendung der Verbindung der Formel (I) als Fixateur.

Verbindungen der Formel (I) sind aufgrund ihres Geruchsprofils besonders geeignet zum Vermitteln, Modifizieren oder Verstärken einer Maiglöckchen-Geruchsnote. Darüber hinaus hat sich gezeigt, dass Verbindungen der Formel (I) im Zusammenwirken mit einem oder mehreren weiteren Riechstoffen, die keine Verbindungen der Formel (I) sind, insbesondere auch mit einem oder mehreren weiteren Riechstoffen, die ihrerseits eine Maiglöckchen-Note aufweisen, in der Lage sind, weitere für parfümistische Zwecke erwünschte Geruchsnoten und Eindrücke hervorzurufen bzw. zu verstärken, insbesondere Geruchsnoten ausgewählt aus der Gruppe bestehend aus blumig (siehe Beispiele 4, 5 und 7), fruchtig (siehe Beispiel 10), wässrig (siehe Beispiele 5 und 10), grün (siehe Beispiele 8 und 9) und aldehydig (siehe Beispiel 6) und/oder Eindrücke ausgewählt aus der Gruppe bestehend aus natürlich (siehe Beispiele 4, 6-8), frisch (siehe Beispiele 5 und 11), pflegend (siehe Beispiel 5), sanft (siehe Beispiel 9), warm (siehe Beispiel 9), komplex und strahlend.

Daher ist erfindungsgemäß eine Verwendung bevorzugt, wobei
eine oder mehrere Verbindungen der Formel (I) wie oben definiert verwendet werden zum Vermitteln, Modifizieren oder Verstärken einer Maiglöckchen-Geruchsnote und
(i) einer, mehrerer oder sämtlicher Geruchsnoten ausgewählt aus der Gruppe bestehend aus blumig, fruchtig, wässrig, grün und aldehydig
   und/oder
(ii) eines, mehrerer oder sämtlicher Eindrücke ausgewählt aus der Gruppe bestehend aus natürlich, frisch, pflegend, sanft, warm, komplex und strahlend.

Darüber hinaus hat sich gezeigt, dass bestimmte Verbindungen der Formel (I) hinsichtlich ihrer Geruchs- und/oder Sekundäreigenschaften besondere Vorteile aufweisen. Erfindungsgemäß bevorzugt ist daher eine Verwendung, wobei
die verwendete Verbindung der Formel (I) oder eine, mehrere oder sämtliche der Verbindungen der Formel (I) in der verwendeten Mischung ausgewählt sind aus der Gruppe bestehend aus Verbindungen, in denen
- n = 1 ist, R1 Wasserstoff ist und R2 eine verzweigte gesättigte Alkylgruppe 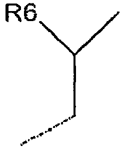 mit insgesamt 4 bis 9 Kohlenstoffatomen ist, in der R6 eine verzweigte oder unverzweigte gesättigte Alkylgruppe mit 1 bis 6 Kohlenstoffatomen ist und die gestrichelte Linie die Bindung darstellt, die die verzweigte gesättigte Alkylgruppe R2 mit dem in Formel (I) benachbarten Kohlenstoffatom verbindet, oder
- n = 1 ist und R1 und R2 gemeinsam mit dem zwischen ihnen angeordneten Kohlenstoffatom einen Cyclohexylring bilden wie oben definiert, R3 und R5 Wasserstoff sind und R4 ausgewählt ist aus der Gruppe bestehend aus Methyl, Ethyl und Isopropyl und tert-Butyl, oder
- n = 0 ist und R1 und R2 gemeinsam mit dem zwischen ihnen angeordneten Kohlenstoffatom einen Phenylring bilden wie oben definiert, R3 und R5 Wasserstoff sind und R4 ausgewählt ist aus der Gruppe bestehend aus Methyl, Ethyl und Isopropyl und tert-Butyl, oder
- n = 1 ist, R1 Methyl ist und R2 eine unverzweigte gesättigte Alkylgruppe mit 4 bis 6 Kohlenstoffatomen ist.

Unter diesen bevorzugt zu verwendenden Verbindungen sind wiederum die Verbindungen bevorzugt: in denen
- n = 1 ist, R1 Wasserstoff ist und R2 eine verzweigte gesättigte Alkylgruppe 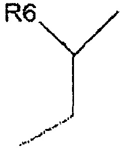 mit insgesamt 4 bis 9 Kohlenstoffatomen ist, in der R6 eine verzweigte oder unverzweigte gesättigte Alkylgruppe mit 1 bis 6 Kohlenstoffatomen ist und die gestrichelte Linie die Bindung darstellt, die die verzweigte gesättigte Alkylgruppe R2 mit dem in Formel (I) benachbarten Kohlenstoffatom verbindet, oder
- n = 1 ist, R1 Methyl ist und R2 eine unverzweigte gesättigte Alkylgruppe mit 4 bis 6 Kohlenstoffatomen ist.

Erfindungsgemäß ganz besonders bevorzugt ist es, dass die Verbindung der Formel (I) oder eine, mehrere oder sämtliche der Verbindungen der Formel (I) in der Mischung ausgewählt sind aus der Gruppe bestehend aus
- 2-(3-Methylbutoxy)acetaldehyd (Formel (Ia)) (2-Isopentyloxyacetaldehyd) 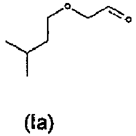
- 2-(4-Isopropylcyclohexyloxy)aretaldehyd (Formel (Ib)) 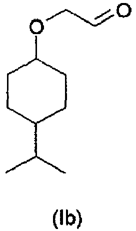
- 2-(4-Isopropylphenoxy)acetaldehyd (Formel (Ic)) 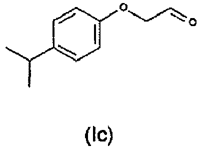
- 2-(3,5,5-Trimethylhexoxy)acetaldehyd (Formel (Id)) 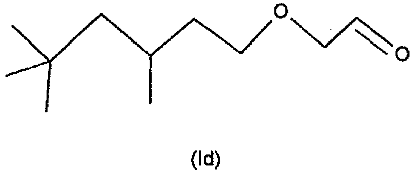
- 2-[(1-Methylheptyl)oxy]acetaldehyd (Formel (Ie))

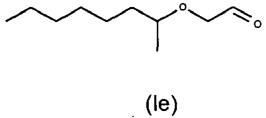

Die Verbindung der Formel (la) wie oben definiert zeichnet sich aus durch ein Geruchsprofil umfassend die Geruchsnoten Grün, Blüten (insbesondere Maiglöckchen und Jasmin), Frucht (insbesondere Banane) und die Eindrücke natürlich und frisch.

Die Verbindung der Formel (Ib) wie oben definiert zeichnet sich aus durch ein Geruchsprofil umfassend die Geruchsnoten Aldehyd, Blüte, insbesondere Maiglöckchen (in einer dem Geruch von 3-(4-Tertbutylphenyl)-2-methylpropanal (Lilial) besonders ähnlichen Komplexität), grün und pudrig sowie die Eindrücke warm und sanft. Die Verbindung der Formel (Ib) ist erfindungsgemäß besonders bevorzugt, da ihr Geruchsprofil alle wesentlichen Geruchsaspekte der Verbindung 3-(4-Tertbutylphenyl)-2-methylpropanal, darunter insbesondere auch die kaum durch andere Riechstoffe nachzubildende pudrige Geruchsnote des Lilial, umfasst. Somit ist diese Verbindung besonders gut geeignet, 3-(4-Tertbutylphenyl)-2-methylpropanal zu ersetzen.

Die Verbindung der Formel (Ic) wie oben definiert zeichnet sich aus durch ein Geruchsprofil umfassend die Geruchsnoten Blüte, insbesondere Maiglöckchen, grün, wässrig (aquatisch).

Die Verbindung der Formel (Id) wie oben definiert zeichnet sich aus durch die Geruchsnote Blüte, insbesondere Maiglöckchen.

Die Verbindung der Formel (Ie) wie oben definiert zeichnet sich aus durch ein Geruchsprofil umfassend die Geruchsnoten aldehydig und Blüte (insbesondere Maiglöckchen).

Einzelne Verbindungen der Formel (I) wurden zwar im Stand der Technik bereits erwähnt, jedoch wurde ihre besondere Eignung zum Vermitteln einer Maiglöckchen-Geruchsnote dabei nicht erkannt.

In der nachstehenden Tabelle sind solche Verbindungen aufgelistet, die an sich bereits bekannt sind, ohne dass ihre besondere Eignung zum Vermitteln einer Mai-glöckchenglöckchen-Geruchsnote bekannt geworden ist. Die Verwendung dieser Verbindungen ist erfindungsgemäß regelmäßig nicht in besonderer Weise bevorzugt, eine Ausnahme bilden insoweit die oben definierten Verbindungen der Formeln (la), (Ic) bzw. (Ie).

| **Nr.** | **Struktur** | **Name** | **Literaturstelle bzw. Hersteller** |
|---|---|---|---|
| 1 | 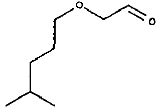 | 2-Isohexyloxyacetaldehyd | Hersteller: Ukrorgsyntez Ltd. |
| 2 | 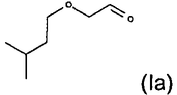 | 2-(3-Methylbutoxy)acetaldehyd (2-Isopentyloxyacetaldehyd) | Hersteller: Ukrorgsyntez Ltd. |
| 3 | 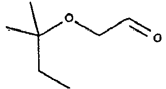 | 2-(1,1-Dimethyl-propoxy)acetaldehyd | Hersteller: Ukrorgsyntez Ltd. |
| 4 | 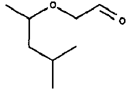 | 2-(1,3-Dimethyl-butoxy)acetaldehyd | Hersteller: Ukrorgsyntez Ltd. |
| 5 | 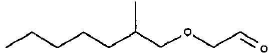 | 2-[(2-Methylheptyl)oxy]-acetaldehyd | *Ann. Chim.* **1934,** *11*, 439-514 |
| 6 | 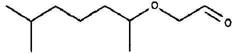 | 2-[(1,5-Dimethylhexyl)oxy]-acetaldehyd | WO2008092678 |
| 7 | 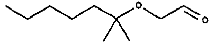 | 2-(1,1-Dimethylhexoxy)-acetaldehyd | *Koryo* **1974,** *107*, 33-37 |
| 8 | 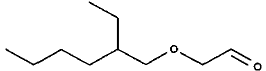 | 2-[(2-Ethylhexyl)oxy]-acetaldehyd | Koryo 1974, 107, 33-37 |
| 9 | 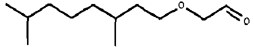 | 2-[(3,7-Dimethyloctyl)oxy]-acetaldehyd | JP 56013701 |
| 10 | 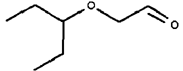 | 2-(1-Ethylpropoxy)acetaldehyd | CN 102180821 |
| | | | Eur. J. Org. Chem. 2011, 26, 5031-5038 |
| | | | Chem. Eur. J. 2010, 16, 12616-12626 |
| | | | Angew. Chem., Int. Ed. 2010, 49, 4656-4660 CN 101735140 |
| | | | Kagaku to Seibutsu 2010, 48, 156-157 |
| | | | WO 2009145263 |
| | | | Angew. Chem., Int. Ed. 2009, 48, 1304-1307 |
| 11 | 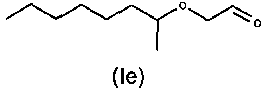 | 2-[(1-Methylheptyl)oxy]-acetaldehyd | Koryo 1974, 107, 33-37 Ann. Chim. 1934, 11, 439-514 |
| | | | Comptes Rendus 1933, 196, 2013 |
| 12 | 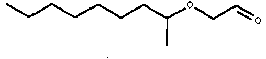 | 2-[(1-Methyloctyl)oxy]acetaldehyd | Synthesis 1979, 202-204 |
| 13 | 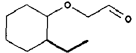 | 2-(2-Ethylcyclohexoxy)-acetaldehyd | Hersteller: Ukrorgsyntez Ltd. |
| 14 | 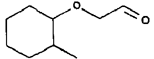 | 2-(2-Methylcyclohexoxy)-acetaldehyd | Ann. Chim. 1934, 11, 439-514 Compt. Rend. 1933, 196, 1508-1510 |
| 15 | 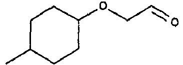 | 2-(4-Methylcyclohexoxy)-acetaldehyd | JP 60190461 Ann. Chim. 1934, 11, 439-514 Compt. Rend. 1933, 196, 1508-1510 |
| 16 | 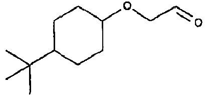 | 2-(4-*tert*Butylcyclohexoxy)-acetaldehyd | |
| 17 | 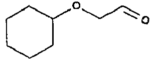 | 2-(Cyclohexoxy)acetaldehyd | Science of synthesis 2007, 25, 17-23 |
| | | | US6221865 |
| | | | WO9850030 |
| | | | JP 60190461 |
| | | | Synthesis 1979, 202-204 |
| | | | Ann. Chim. 1934, 11, 439-514 |
| | | | Compt. Rend. 1933, 196, 1508-1510 |
| 18 | 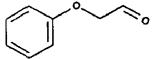 | 2-Phenoxyacetaldehyd | Ann. Chim. 1934, 11, 439-514 |
| 19 | 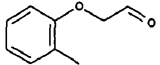 | 2-(2-Methylphenoxy)acetaldehyd | Russian Journal of Organic Chemistry 2010, 46, 1017-1020 |
| | | | WO 2004058755 |
| | | | Monatshefte für Chemie 1996, 127, 167-172 |
| | | | Bull. Soc. Chem. Fr. 1991, 81-86 |
| | | | EP 169581 |
| | | | Meddelelser fra Norsk Farmaceutisk Selskap 1959, 21, 97-104 |
| | | | Bull. Soc. Chem. Fr. 1953, 479-483 |
| 20 | 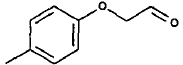 | 2-(4-Methylphenoxy)acetaldehyd | Monatshefte für Chemie 1996, 127, 167-172 |
| 21 | 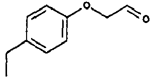 | 2-(4-Ethylphenoxy)acetaldehyd | Hersteller: Ambinter |
| 22 | 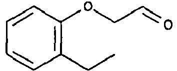 | 2-(2-Ethylphenoxy)acetaldehyd | Hersteller: Ambinter |
| 23 | 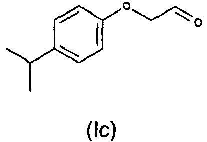 | 2-(4-Isopropyl-phenoxy)acetaldehyd | WO2003033437 |
| 24 | 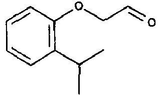 | 2-(2-Isopropylphenoxy)-acetaldehyd | Revista de Farmacia e Bioquimica da Universidade de Sao Paulo 1998, 34, 77-83 |
| | | | Bull. Soc. Chem. Fr. 1991, 81-86 |
| 25 | 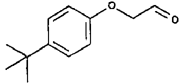 | 2-(4-*tert*Butylphenoxy)-acetaldehyd | Bull. Soc. Chim. Fr. 1929, 45, 1161 |
| | | | WO 2010017986 |
| | | | DE 102008039417 |
| | | | DE 10246329 |
| | | | EP 553623 |
| 26 | 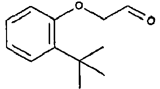 | 2-(2-*tert*Butylphenoxy)-acetaldehyd | Hersteller: Ambinter |
| 27 | 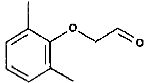 | 2-(2,6-Dimethylphenoxy)-acetaldehyd | WO2009012998 |
| | | | WO2007119984 |
| | | | Monatshefte für Chemie 1996, 127, 167-172 |
| | | | Bull. Soc. Chem. Fr. 1991, 81-86 |
| | | | DE3021958 |
| | | | Helv. Chim. Acta 1953, 36, 489-500 |
| 28 | 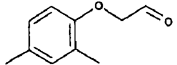 | 2-(2,4-Dimethylphenoxy)-acetaldehyd | Bull. Soc. Chem. Fr. 1991, 81-86 |
| 29 | 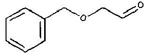 | 2-Benzyloxyacetaldehyd | JP2007246435 |
| | | | Ann. Chim. 1934, 11, 439-514 |
| 30 | 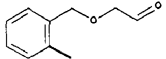 | 2-(*o*-Tolylmethoxy)acetaldehyd | WO2005011697 |
| 31 | 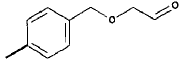 | 2-(*p*-Tolylmethoxy)acetaldehyd | WO2005011697 |
| 32 | 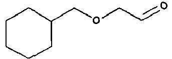 | 2-(Cyclohexylmethoxy)-acetaldehyd | Hersteller: Ukrorgsyntez Ltd. |

Gegenstand der vorliegenden Erfindung ist auch eine Verbindung der Formel (I) wie in Anspruch 6 definiert.

Unter den erfindungsgemäßen Verbindungen der Formel (I) sind die Verbindung der Formel (Ib) wie oben definiert sowie die Verbindung der Formel (Id) wie oben definiert besonders bevorzugt.

Gegenstand der vorliegenden Erfindung ist auch die Verbindung 1-(2,2-Diethoxyethoxy)-4-isopropylcyclohexan (Formel (IIb) 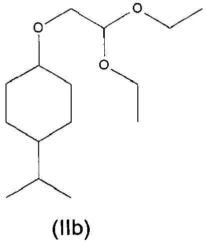 welche eine Vorstufe der Verbindung 2-(4-Isopropylcyclohexyloxy)acetaldehyd (Formel (Ib)) darstellt (siehe Beispiele 1 und 3).

Ein weiterer Aspekt der vorliegenden Erfindung betrifft eine Mischung enthaltend eine oder mehrere Verbindungen der Formel (I) wie oben definiert, vorzugsweise eine oder mehrere Verbindungen der Formel (I) wie oben als bevorzugt charakterisiert
wobei die Mischung
- einen, 2, 3, 4, 5, 6, 7, 8, 9, 10 oder mehr weitere Riechstoffe enthält, die keine Verbindungen der Formel (I) sind,
und/oder
- ein oder mehrere Lösungsmittel enthält, die ausgewählt sind aus der Gruppe bestehend aus den Trialkylcitraten (vorzugsweise Triethylcitrat, Trimethylcitrat, Tripropylcitrat, Triisopropylcitrat, Tributylcitrat, Triisobutylcitrat oder Tri-secbutylcitrat), den Dialkylphthalaten (vorzugsweise Dimethylphthalat, Diethylphthalat oder Dibutylphthalat), Benzylbenzoat, Isopropylmyristat und Dioctyladipat,
und/oder
- ein Produkt ist ausgewählt ist aus der Gruppe bestehend aus Wasch- und Reinigungsmitteln, Hygiene- oder Pflegeprodukten, insbesondere aus dem Bereich der Körper- und Haarpflege, der Kosmetik und des Haushalts, Fine Fragrances (Parfüme), Parfümölen, Air Care-Produkten (insbesondere Kerzen), Lufterfrischern, Mitteln zum Reinigen, Behandeln und/oder Pflegen von Oberflächen und textilen Fasern.

Eine erste Gruppe erfindungsgemäßer Mischungen sind Mischungen bestehend aus oder umfassend
- eine oder mehrere Verbindungen der Formel (I) wie oben definiert, vorzugsweise eine oder mehrere Verbindungen der Formel (I) wie oben als bevorzugt charakterisiert
und
- einen, 2, 3, 4, 5, 6, 7, 8, 9, 10 oder mehr weitere Riechstoffe, die keine Verbindung der Formel (I) sind.

In einer derartigen erfindungsgemäßen Mischung liegt die Gesamtmenge der Verbindungen der Formel (I) wie oben definiert im Bereich von 0,0001 bis 40 Gew.-%, vorzugsweise im Bereich von 0,001 bis 25 Gew.-%, jeweils bezogen auf das Gesamtgewicht der Mischung.

Derartige erfindungsgemäße Mischungen werden nachstehend auch als Riechstoffmischungen bzw. Riechstoffzubereitungen bezeichnet. Eine typische Form von Riechstoffzubereitungen sind Parfümöle.

Insbesondere durch Kombination einer oder mehrerer Verbindungen der Formel (I) wie oben definiert, vorzugsweise in einer der als bevorzugt angegebenen Ausgestaltungen, mit einem oder mehren weiteren Riechstoffen (vorzugsweise mit einer, mehreren oder sämtlichen Geruchsnoten ausgewählt aus der Gruppe bestehend aus blumig, wässrig, fruchtig, grün, aldehydig), die keine Verbindungen der Formel (I) sind, lassen sich Mischungen mit besonders interessanten, natürlichen, neuen und originellen Noten kreieren. Dies gilt insbesondere auch für Kombinationen einer oder mehrerer Verbindungen der Formel (I) mit einem oder mehreren weiteren Riechstoffen, die ihrerseits eine Maiglöckchen-Note aufweisen, aber keine Verbindungen der Formel (I) sind.

Riechstoffe, die im Sinne der obigen Definition als weitere Riechstoffe für den Einsatz in einer erfindungsgemäßem Mischung geeignet sind, finden sich z. B. in S. Arctander, Perfume and Flavor Materials, Vol. I und II, Montclair, N. J. 1969, Eigenverlag, oder K. Bauer et al., Common Fragrance and Flavor Materials, 4th Edition, Wiley-VCH, Weinheim 2001.

Im Einzelnen seien genannt: Extrakte aus natürlichen Rohstoffen wie Etherische Öle, Concretes, Absolues, Resine, Resinoide, Balsame, Tinkturen wie z. B.
Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernnadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepresst; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnussöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierten Inhaltsstoffen;
Einzel-Riechstoffe aus der Gruppe der Kohlenwasserstoffe, wie z.B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole wie z.B. Hexanol; Octanol; 3-Octanol; 2,6-Dimethylheptanol; 2-Methyl-2-heptanol; 2-Methyl-2-octanol; (E)-2-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale wie z.B. Hexanal; Heptanal; Octanal; Nonanal; Decanal; Undecanal; Dodecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxy-propoxy)-(E/Z)-3-hexen;
der aliphatischen Ketone und deren Oxime wie z.B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on;
der aliphatischen schwefelhaltigen Verbindungen wie z.B. 3-Methylthio-hexanol; 3-Methylthiohexylacetat; 3-Mercaptohexanol; 3-Mercaptohexylacetat; 3-Mercapto hexylbutyrat; 3-Acetylthiohexylacetat; 1-Menthen-8-thiol;
der aliphatischen Nitrile wie z.B. 2-Nonensäurenitril; 2-Undecensäurenitril; 2-Tridecensäurenitril; 3,12-Tridecadiensäurenitril; 3,7-Dimethyl-2,6-octadien-säurenitril; 3,7-Dimethyl-6-octensäurenitril;
der Ester von aliphatischen Carbonsäuren wie z.B. (E)- und (Z)-3-Hexenylformiat; Ethylacetoacetat; Isoamylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadien-oat;4-Methyl-2-pentyl-crotonat;
der acyclischen Terpenalkohole wie z. B. Geraniol; Nerol; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
der acyclischen Terpenaldehyde und -ketone wie z. B. Citronellal;; 7-Methoxy-3,7-dimethyloctanal; 2,6,10-Trimethyl-9-undecenal; Geranylaceton; sowie die Dimethyl- und Diethylacetale von Geranial, Neral,
der cyclischen Terpenalkohole wie z. B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate;
der cyclischen Terpenaldehyde und -ketone wie z. B. Menthon; Isomenthon; 8-Mercaptomenthan-3-on; Carvon; Campher; Fenchon; alpha-Ionon; beta-Ionon, alpha-n-Methylionon; beta-n-Methylionon; alpha-Isomethylionon; beta-Isomethyl-ionon; alpha-Iron; beta-Damascenon; 1-(2,4,4-Trimethyl-2-cyclohexen-1-yl)-2-buten-1-on; 1,3,4,6,7,8a-Hexahydro-1,1,5,5-tetramethyl-2H-2,4a-methano-naphthalen-8(5H)-on;2-Methyl-4-(2,6,6-trimethyl-1-cyclohexen-1-yl)-2-butenal; Nootkaton; Dihydronootkaton; 4,6,8-Megastigmatrien-3-on; alpha-Sinensal; beta-Sinensal; acetyliertes Cedernholzöl (Methylcedrylketon);
der cyclischen Alkohole wie z.B. 4-tert.-Butylcyclohexanol; 3,3,5-Trimethylcyclohexanol; 3-Isocamphylcyclohexanol; 2,6,9-Trimethyl-Z2,Z5,E9-cyclododecatrien-1-ol; 2-isobutyl-4-methyltetrahydro-2H-pyran-4-ol;
der cycloaliphatischen Alkohole wie z.B. alpha,3,3-Trimethylcyclohexylmethanol;1-(4-Isopropylcyclohexyl)ethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z.B. Cineol; Cedrylmethylether; Cyclododecylmethylether;1,1-Dimethoxycyclododecan; (Ethoxymethoxy)cyclo-dodecan; alpha-Cedrenepoxid; 3a,6,6,9a-Tetramethyldodecahydro-naphtho[2,1-b]furan; 3a-Ethyl-6,6,9a-trimethyldodecahydronaphtho[2,1b]furan; 1,5,9-Tri-methyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen und makrocyclischen Ketone wie z.B. 4-tert.-Butylcyclohexanon; 2,2,5-Trimethyl-5-pentylcyclopentanon; 2-Heptylcyclopentanon; 2-Pentyl-cyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopenta-decanon; 4-(1-Ethoxyvinyl)-3,3,5,5-tetramethylcyclohexanon; 4-tert.-Pentylcyclo-hexanon; 5-Cyclohexadecen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon;
der cycloaliphatischen Aldehyde wie z.B. 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z. B. 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z.B. 2-tert-Butylcyclohexylacetat; 4-tert-Butylcyclohexylacetat; 2-tert-Pentylcyclohexylacetat; 4-tert-Pentyl-cyclo-hexyl-acetat; 3,3,5-Trimethylcyclohexylacetat; Decahydro-2-naphthylacetat;2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexa-hydro-5, bzw. 6-indenylacetat; 4,7-Methano-3a,4,5,6,7,7a-hexahydro-5, bzw. 6-indenyl-propionat; 4,7-Methano-3a,4,5,6,7,7a-hexahydro-5, bzw. 6-inden-ylisobutyrat; 4,7-Methanooctahydro-5, bzw. 6-indenylacetat;
der Ester cycloaliphatischer Alkohole wie z.B.1-Cyclohexylethylcrotonat;
der Ester cycloaliphatischer Carbonsäuren wie z. B. Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexen-carboxylat; Ethyl-2-methyl-1,3-dioxolan-2-acetat;
der araliphatischen Alkohole wie z.B. Benzylalkohol; 1-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenyl-propanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenyl-ethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether wie z.B. 2-Phenylethylmethylether; 2-Phenylethyl-isoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetra-hydroindeno[1,2-d]-m-dioxin; 4,4a,5,9b-Tetrahydro-2,4-dimethylindeno[1,2-d]-m-dioxin;
der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-isobutylphenyl)propanal; 3-(4-tert.-Butylphenyl)propanal; Zimtaldehyd; alpha-Butylzimtaldehyd; alpha-Hexylzimtaldehyd; 3-Methyl-5-phenylpentanal; 4-Methoxybenzaldehyd; 4-Hydroxy-3-methoxybenz-aldehyd; 4-Hydroxy-3-ethoxybenzaidehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4-Methylacetophenon; 4-Methoxyacetophenon; 4-tert.-Butyl-2,6-dimethylaceto-phenon; 4-Phenyl-2-butanon; 4-(4-Hydroxyphenyl)-2-butanon; 1-(2-Naphtha-lenyl)ethanon;2-Benzofuranylethanon;(3-Methyl-2-benzofuranyl)ethanon; Benzophenon; 1,1,2,3,3,6-Hexamethyl-5-indanylmethylketon; 6-tert-Butyl-1,1-di-methyl-4-indanylmethylketon; 1-[2,3-dihydro-1,1,2,6-tetramethyl-3-(1-methylethyl) -1H-5-indenyl]ethanon; 5',6',7',8'-Tetrahydro-3',5',5',6',8',8'-hexamethyl-2-aceto-naphthon;
der aromatischen und araliphatischen Carbonsäuren und deren Ester wie z.B. Benzoesäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Hexylsalicylat; Cyclohexylsalicylat;Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentan-säurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butyl-phenyl)propanal oder 2,4-Dimethyl-3-cyclohexencarbaldehyd; 6-Isopropyl-chinolin; 6-Isobutylchinolin; 6-sec.-Butylchinolin;2-(3-Phenylpropyl)pyridin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenylmethylether; Isoeugenol; lsoeugenylmethylether; Thymol; Carvacrol; Diphenylether; beta-Naphthylmethylether; beta-Naphthylethylether; beta-Naphthyliso-butylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-0ctanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid;4-Methyl-1,4-decanolid; 1,15-Pentadecanolid; 1,16-Hexadeca-nolid; 9-Hexadecen-1,16-olid; 10-Oxa-1,16-hexadecanolid, 11-Oxa-1,16-hexa-decanolid; 12-Oxa-1,16-hexadecanolid; Ethylen-1,12-dodecandioat; Ethylen-1,13-tridecandioat; 2,3-Dihydrocumarin; Octahydrocumarin.

Neben den oben genannten Riechstoffen enthält eine derartige erfindungsgemäße Mischung gegebenenfalls weitere Bestandteile, die selbst keine Riechstoffe im Sinne der obenstehenden Definition sind wie z.B. Lösungsmittel, Lösungsvermittler, Emulgatoren, Stabilisatoren, Radikalfänger.

In einer bevorzugten Ausführungsform umfasst eine erfindungsgemäße Mischung, insbesondere in Form eines Parfümöls, einen, 2, 3 oder mehr weitere Riechstoffe mit einer Maiglöckchen-Geruchsnote und optional einer, mehreren oder sämtlichen Geruchsnoten ausgewählt aus der Gruppe bestehend aus blumig, fruchtig, grün, wässrig und aldehydig. Entsprechende Riechstoffe sind dem Fachmann bekannt.

Besonders bevorzugt ist eine erfindungsgemäße Riechstoffzubereitung, in welcher
(i) die Menge der Verbindung(en) der Formel (I) ausreicht, um der Riechstoffzubereitung eine Maiglöckchen-Geruchsnote und optional eine, mehrere oder sämtliche Geruchsnoten ausgewählt aus der Gruppe bestehend aus blumig, fruchtig, grün, wässrig und aldehydig vermitteln,
   und/oder
(ii) ein, mehrere oder sämtliche weitere Riechstoffe der Riechstoffzubereitung eine Maiglöckchen-Geruchsnote und optional eine, mehrere oder sämtliche Geruchsnoten ausgewählt aus der Gruppe bestehend aus blumig, fruchtig, grün, wässrig und aldehydig vermitteln, wobei die Menge der Verbindung(en) der Formel (I) ausreicht, um im Vergleich zu einer ansonsten identisch zusammengesetzten Vergleichs-Riechstoffzubereitung ohne Verbindung(en) der Formel (I) die Maiglöckchen-Geruchsnote und optional eine, mehrere oder sämtliche der Geruchsnoten blumig, fruchtig, grün, wässrig und aldehydig zu verstärken,
   und/oder
(iii) die Menge der Verbindung(en) der Formel (I) ausreicht, um der Riechstoffzubereitung einen, mehrere oder sämtliche Eindrücke ausgewählt aus der Gruppe bestehend aus natürlich, frisch, pflegend, sanft, warm, komplex und strahlend zu vermitteln
   und/oder
(iv) die Menge der Verbindung(en) der Formel (I) ausreicht, um einen, mehrere oder sämtliche Eindrücke ausgewählt aus der Gruppe bestehend aus natürlich, frisch, pflegend, sanft, warm, komplex und strahlend im Vergleich zu einer ansonsten identisch zusammengesetzten Vergleichs-Riechstoffzubereitung ohne Verbindung(en) der Formel (I) zu modifizieren und/oder zu verstärken.

Erfindungsgemäße Riechstoffzubereitungen sind z.B. erhältlich durch ein Verfahren umfassend den Schritt des Mischens von
- einer oder mehreren Verbindungen der Formel (I) wie oben definiert
und
- einem, 2, 3, 4, 5, 6, 7, 8, 9, 10 oder mehren weiteren Riechstoffen, die keine Verbindung der Formel (I) sind,
- sowie gegebenenfalls weiteren Bestandteilen, die keine Riechstoffe sind.

Eine zweite Gruppe erfindungsgemäßer Mischungen sind Mischungen bestehend aus oder umfassend
- eine oder mehrere Verbindungen der Formel (I) wie oben definiert, vorzugsweise eine oder mehrere Verbindungen der Formel (I) wie oben als bevorzugt charakterisiert
und
- ein oder mehrere Lösungsmittel, die ausgewählt sind aus der Gruppe bestehend aus den Trialkylcitraten (vorzugsweise Triethylcitrat, Trimethylcitrat, Tripropylcitrat, Triisopropylcitrat, Tributylcitrat, Triisobutylcitrat oder Tri-sec-butylcitrat), den Dialkylphthalaten (vorzugsweise Dimethylphthalat, Diethylphthalat oder Dibutylphthalat), Benzylbenzoat, Isopropylmyristat und Dioctyladipat.

In einer solchen erfindungsgemäßen Mischung liegt die Gesamtmenge der Verbindungen der Formel (I) wie oben definiert im Bereich von 0,0001 bis 40 Gew.-%, vorzugsweise im Bereich von 0,001 bis 25 Gew.-%, jeweils bezogen auf das Gesamtgewicht der Mischung.

Lösungsmittel aus der Gruppe bestehend aus den Trialkylcitraten, den Dialkylphthalaten, Benzylbenzoat, Isopropylmyristat und Dioctyladipat sind bevorzugte Lösungsmittel für die Verbindung der Formel (I), da sie im Gegensatz zu anderen, herkömmlicherweise in parfümierten Produkten (siehe unten) eingesetzten Lösungsmitteln wie z.B. Dipropylenglycol nicht zu einer Verringerung der Intensität der Maiglöckchen-Note führen (siehe auch Beispiel 27).

Bevorzugt sind Mischungen, in denen das oder eines der Lösungsmittel ausgewählt ist aus der Gruppe bestehend aus Triethylcitrat, Diethylphthalat, Benzylbenzoat, Isopropylmyristat und Dioctyladipat.

Mischungen bestehend aus (i) einem Lösungsmittel aus der Gruppe bestehend aus Triethylcitrat, Dimethylphthalat, Diethylphthalat, Dibutylphthalat, Benzylbenzoat und Isopropylmyristat und (ii) einer oder beiden Verbindungen aus der Gruppe bestehend aus Phenoxyacetaldehyd und p-Methylphenoxyacetaldehyd sind dabei nicht bevorzugt.

Weiterhin ist es bevorzugt, dass eine erfindungsgemäße Mischung, die (i) eine oder beide Verbindungen aus der Gruppe bestehend aus Phenoxyacetaldehyd und p-Methylphenoxyacetaldehyd sowie (ii) ein Lösungsmittel aus der Gruppe bestehend aus Triethylcitrat, Dimethylphthalat, Diethylphthalat, Dibutylphthalat, Benzylbenzoat und Isopropylmyristat enthält, daneben eine oder mehrere weitere Verbindungen der Formel (I) (wie oben definiert) und/oder ein oder mehrere weitere Lösungsmittel aus der Gruppe bestehend aus den Trialkylcitraten (mit Ausnahme von Triethylcitrat) und Dioctyladipat enthält.

Für eine erfindungsgemäße Mischung, die eine oder mehrere Verbindungen der Formel (I) und ein oder mehrere Lösungsmittel aus der Gruppe bestehend aus den Trialkylcitraten, den Dialkylphthalaten, Benzylbenzoat, Isopropylmyristat und Dioctyladipat enthält, ist es außerdem bevorzugt, dass
- die Mischung kein Lösungsmittel aus der Gruppe bestehend aus Triethylcitrat, Dimethylphthalat, Diethylphthalat, Dibutylphthalat, Benzylbenzoat und Isopropylmyristat enthält, wenn die Mischung eine oder beide Verbindungen aus der Gruppe bestehend aus Phenoxyacetaldehyd und p-Methylphenoxyacetaldehyd enthält
und
- die Mischung keine Verbindung aus der Gruppe bestehend aus Phenoxyacetaldehyd und p-Methylphenoxyacetaldehyd enthält, wenn die Mischung ein Lösungsmittel aus der Gruppe bestehend aus Triethylcitrat, Dimethylphthalat, Diethylphthalat, Dibutylphthalat, Benzylbenzoat und Isopropylmyristat enthält.

Es ist am meisten bevorzugt, dass eine erfindungsgemäßen Mischung, die eine oder mehrere Verbindungen der Formel (I) und ein oder mehrere Lösungsmittel aus der Gruppe bestehend aus den Trialkylcitraten, den Dialkylphthalaten, Benzylbenzoat, Isopropylmyristat und Dioctyladipat enthält, keine Verbindung aus der Gruppe bestehend aus Phenoxyacetaldehyd und p-Methylphenoxyacetaldehyd enthält.

Die erfindungsgemäß zu verwendenden Verbindungen der Formel (I) sowie erfindungsgemäße Mischungen, die eine oder mehrere Verbindungen der Formel (I) und ein oder mehrere Lösungsmittel aus der Gruppe der Trialkylcitrate enthalten, sowie erfindungsgemäße Riechstoffzubereitungen wie oben definiert werden insbesondere zur Herstellung parfümierter Produkte (parfümierter Artikel) eingesetzt. Derartige parfümierte Produkte stellen somit eine dritte Gruppe erfindungsgemäßer Mischungen dar. Hierbei ist die erfindungsgemäße Mischung vorzugsweise ein parfümiertes Produkt ausgewählt aus der Gruppe bestehend aus Wasch- und Reinigungsmitteln, Hygiene- oder Pflegeprodukten, insbesondere aus dem Bereich der Körper- und Haarpflege, der Kosmetik und des Haushalts, Fine Fragrances (Parfüme), Air Care-Produkten (insbesondere Kerzen), Lufterfrischern, Mitteln zum Reinigen, Behandeln und/oder Pflegen von Oberflächen und textilen Fasern.

In einer erfindungsgemäßen Mischung in Form eines Produkts ausgewählt aus der Gruppe bestehend aus Wasch- und Reinigungsmitteln, Hygiene- oder Pflegeprodukten, insbesondere aus dem Bereich der Körper- und Haarpflege, der Kosmetik und des Haushalts, Fine Fragrances (Parfüme), Air Care-Produkten (insbesondere Kerzen), Lufterfrischem, Mitteln zum Reinigen, Behandeln und/oder Pflegen von Oberflächen und textilen Fasern liegt die Gesamtmenge der Verbindungen der Formel (I) wie oben definiert im Bereich von 0,0001 bis 5 Gew.-%, vorzugsweise im Bereich von 0,001 bis 2,5 Gew.-%, jeweils bezogen auf das Gesamtgewicht des Produkts.

Hinsichtlich bevorzugter Ausgestaltungen gelten die obigen Ausführungen entsprechend.

Bevorzugte erfindungsgemäße Produkte sind ausgewählt aus der Gruppe bestehend aus:
Parfüm-Extraits, Eau de Parfums, Eau de Toilettes, Rasierwässer, Eau de Colognes, Pre-shave-Produkte, Splash-Colognes, parfümierte Erfrischungstücher, Parfüms für saure, alkalische und neutrale Reinigungsmittel, Waschmittel, Waschtabletten, Desinfektionsmitteln, sowie von Luftverbesserern, Aerosolsprays, Wachse und Polituren, sowie Körperpflegemitteln, Badeölen, kosmetischen Emulsionen, wie z.B. Hautcremes- und-lotionen, Sonnenschutzcremes- und -lotionen, After-sun-cremes und -lotionen, Handcremes und -lotionen, Fußcremes und -lotionen, Enthaarungscremes und -lotionen, Aftershave-Cremes und -lotionen, Bräunungscremes und -lotionen, Haarpflegeprodukten wie z.B. Haarsprays, Haargelen, festigende Haarlotionen, Haarspülungen, Haarfärbemitteln, Haarverformungsmitteln und Haarglättungsmitteln, Haarwässern, Haarcremes und - lotionen, Deodorantien und Antiperspirantiens, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repellentien, Treibstoffen.

Erfindungsgemäße Riechstoffzubereiturigen enthaltend die Verbindung der Formel (I) oder eine wie oben definierte erfindungsgemäße Mischung können allgemein (z.B. in konzentrierter Form, in Lösungen oder in unten beschriebener modifizierter Form) verwendet werden für die Herstellung von z.B. Parfüm-Extraits, Eau de Parfums, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigem, Fensterglasreinigern, Geschirrspülmittel, Bad- und Sanitärreinigem, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z.B. Hautcremes- und -lotionen, Gesichtscremes und -lotionen, Sonnenschutzcremes und -lotionen, After-sun-cremes und-lotionen, Handcremes und -lotionen, Fußcremes und -lotionen, Enthaarungscremes und-lotionen, After-shave-Cremes und -lotionen, Bräunungscremes und -lotionen, Haarpflegeprodukten wie z.B. Haarsprays, Haargelen, festigen Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repellentien, Treibstoffen.

Bevorzugte erfindungsgemäße parfümierte Produkte sind Produkte ausgewählt aus der Gruppe bestehend aus Wasch- und Reinigungsmitteln, Hygiene- oder Pflegeprodukten, insbesondere aus dem Bereich der Körper und Haarpflege, der Kosmetik und des Haushalts, Fine Fragrances (Parfüme), Air Care-Produkten (insbesondere Kerzen), Lufterfrischern.

Die erfindungsgemäßen zuvor genannten Riechstoffzubereitungen bzw. die erfindungsgemäß in den entsprechenden Produkten einzusetzenden Riechstoffzubereitungen können in flüssiger Form, unverdünnt oder mit einem Lösungsmittel verdünnt für Parfümierungen eingesetzt werden. Geeignete Lösungsmittel hierfür sind z.B. Ethanol, Isopropanol, Diethylenglycolmonoethylether, Glycerin, Propylenglycol, 1,2-Butylenglycol, Dipropylenglycoi, Diethylphthalat, Trialkylcitrate, insbesondere Triethylcitrat, Isopropylmyristat usw. Für die explizit genannten Lösungsmittel gilt, dass diese im Rahmen des vorliegenden Textes im Falle des Vorhandenseins eigener olfaktorischer Eigenschaften ausschließlich dem Bestandteil "Lösungsmittel" und nicht den "Riechstoffen" zuzuordnen sind.

Die in den erfindungsgemäßen parfümierten Produkten enthaltene(n) Verbindung(en) der Formel (I) bzw. die in den erfindungsgemäßen parfümierten Produkten enthaltene erfindungsgemäße Mischung der ersten oder zweiten Gruppe (wie oben definiert) kann dabei in einer bevorzugten Ausführungsform an einem Trägerstoff absorbiert sein, der sowohl für eine feine Verteilung der Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer und Cellulose-basierende Stoffe sein.

Die in den erfindungsgemäßen parfümierten Produkten enthaltene(n) Verbindung(en) der Formel (I) bzw. die in den erfindungsgemäßen parfümierten Produkten enthaltene erfindungsgemäße Mischung der ersten oder zweiten Gruppe (wie oben definiert) kann auch mikroverkapselt, sprühgetrocknet, als Einschluss-Komplexe oder als Extrusions-Produkte vorliegen und in dieser Form dem zu parfümierenden Produkt, bzw. Artikel, hinzugefügt werden.

Gegebenenfalls können die Eigenschaften der derart modifizierten Mischungen durch sogenanntes "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden.

Die Mikroverkapselung der Riechstoffzubereitungen kann beispielsweise durch das sogenannte Koazervationsverfahren mit Hilfe von Kapselmaterialien, z.B. aus polyurethan-artigen Stoffen oder Weichgelatine, erfolgen. Die sprühgetrockneten Parfümöle können beispielsweise durch Sprühtrocknung einer das Parfümöl enthaltenden Emulsion bzw. Dispersion hergestellt werden, wobei als Trägerstoffe modifizierte Stärken, Proteine, Dextrin und pflanzliche Gummen verwendet werden können. Einschluss-Komplexe können z.B. durch Eintragen von Dispersionen von der Riechstoffzubereitung und Cyclodextrinen oder Harnstoffderivaten in ein geeignetes Lösungsmittel, z.B. Wasser, hergestellt werden. Extrusions-Produkte können durch Verschmelzen der Riechstoffzubereitung mit einem geeigneten wachsartigen Stoff und durch Extrusion mit nachfolgender Erstarrung, ggf. in einem geeigneten Lösungsmittel, z.B. Isopropanol, erfolgen.

Die erfindungsgemäß zu verwendende(n) Verbindung(en) der Formel (I) bzw. die erfindungsgemäßen Mischungen der ersten oder zweiten Gruppe (wie oben definiert) können demnach, wie bereits erwähnt, in konzentrierter Form, in Lösungen oder in oben beschriebener modifizierter Form für die Herstellung der entsprechenden erfindungsgemäßen Produkte verwendet werden.

Zutaten, mit denen die erfindungsgemäße Verbindung der Formel (I) oder die erfindungsgemäße Mischung der ersten oder zweiten Gruppe (wie oben definiert) vorzugsweise kombiniert werden kann, sind beispielsweise: Konservierungsmittel, Abrasiva, Antiakne-Mittel, Mittel gegen Hautalterung, antibakterielle Mittel, Anticellulitis-Mittel, Antischuppen-Mittel, entzündungshemmende Mittel, irritationsverhindernde Mittel, irritationshemmende Mittel, antimikrobielle Mittel, Antioxidantien, Adstringentien, schweißhemmende Mittel, antiseptische Mittel, Antistatika, Binder, Puffer, Trägermaterialien, Chelatbildnder, Zellstimulantien, reinigende Mittel, pflegende Mittel, Enthaarungsmittel, oberflächenaktive Substanzen, deodorierende Mittel, Antiperspirantien, Weichmacher, Emulgatoren, Enzyme, ätherische Öle, Fasern, Filmbildner, Fixateure, Schaumbildner, Schaumstabilisatoren, Substanzen zum Verhindern des Schäumens, Schaumbooster, Fungizide, gelierende Mittel, gelbildende Mittel, Haarpflegemittel, Haarverformungsmittel, Haarglättungsmittel, feuchtigkeitsspendende Mittel, anfeuchtende Substanzen, feuchthaltende Substanzen, bleichende Mittel, stärkende Mittel, fleckenentfernende Mittel, optisch aufhellende Mittel, Imprägnierende Mittel, schmutzabweisende Mittel, reibungsverringemde Mittel, Gleitmittel, Feuchtigkeitscremes, Salben, Trübungsmittel, plastifizierende Mittel, deckfähige Mittel, Politur, Glanzmittel, Polymere, Pulver, Proteine, rückfettende Mittel, abschleifende Mittel, Silicone, hautberuhigende Mittel, hautreinigende Mittel, hautpflegende Mittel, hautheilende Mittel, Hautaufhellungsmittel, hautschützende Mittel, hauterweichende Mittel, kühlende Mittel, hautkühlende Mittel, wärmende Mittel, hautwärmende Mittel, Stabilisatoren, UV-absorbierende Mittel, UV-Filter, Waschmittel, Weichspüler, suspendierende Mittel, Hautbräunungsmittel, Verdickungsmittel, Vitamine, Öle, Wachse, Fette, Phospholipide, gesättigte Fettsäuren, ein- oder mehrfach ungesättigte Fettsäuren, α-Hydroxysäuren, Polyhydroxyfettsäuren, Verflüssiger, Farbstoffe, farbschützende Mittel, Pigmente, Antikorrosiva, Aromen, Geschmackstoffe, Polyole, Tenside, Elektrolyte, organische Lösungsmittel oder Silikonderivate.

Ein weiterer Aspekt der vorliegenden Erfindung betrifft ein Verfahren zum Vermitteln, und/oder Verstärken einer Maiglöckchen-Geruchsnote und optional einer oder mehrerer weiterer Geruchsnoten und/oder eines oder mehrerer Eindrücke. Ein erfindungsgemäßes Verfahren zum Vermitteln und/oder Verstärken
(i) einer Maiglöckchen-Geruchsnote
   sowie (ii) gegebenenfalls
(ii)a) einer, mehrerer oder sämtlicher Geruchsnoten ausgewählt aus der Gruppe bestehend aus blumig, fruchtig, grün, wässrig und aldehydig
   und/oder
(ii)b) eines, mehrerer oder sämtlicher Eindrücke ausgewählt aus der Gruppe bestehend aus natürlich, frisch, pflegend, sanft, warm, komplex und strahlend;
umfasst den folgenden Schritt:
Mischen oder in Kontakt bringen
   - einer oder mehrerer Verbindungen der Formel (I) wie oben definiert, oder
   - einer erfindungsgemäßen Mischung wie oben definiert
vorzugsweise in einer sensorisch wirksamen Menge mit einem Erzeugnis.

Hinsichtlich bevorzugter Ausgestaltungen gelten die obigen Ausführungen entsprechend.

Das vorstehend beschriebene erfindungsgemäße Verfahren ist auch geeignet im Rahmen der Herstellung einer erfindungsgemäßen Mischung in Form eines Produkts wie oben beschrieben, wobei als Ergebnis des Schrittes Mischen oder in Kontakt bringen
- einer oder mehrerer Verbindungen der Formel (I) wie oben definiert, oder
- einer erfindungsgemäßen Mischung wie oben definiert
vorzugsweise in einer sensorisch wirksamen Menge mit einem Erzeugnis das erfindungsgemäße parfümierte Produkt gebildet wird. Unter "Erzeugnis" ist in diesem Fall die Gesamtheit der Bestandteile des erfindungsgemäßen Produkts zu verstehen mit Ausnahme der eingesetzten Verbindung der Formel (I), der eingesetzten erfindungsgemäßen Mischung bzw. der eingesetzten erfindungsgemäßen Riechstoffzubereitung.

Bei dem erfindungsgemäßen Verfahren erweist sich die hohe Substantivität der erfindungsgemäß zu verwendenden Verbindungen der Formel (I) als vorteilhaft, denn je höher die Substantivität eines Riechstoffs, desto geringer ist die zum Aufrechterhalten eines Geruchs über eine bestimmte Zeit benötigte Einsatzmenge des Riechstoffs.

Ein weiterer Aspekt der vorliegenden Erfindung betrifft ein Verfahren zum Versehen von Haut, Haaren, Oberflächen oder textilen Fasern mit einer Maiglöckchen-Geruchsnote. Ein erfindungsgemäßes Verfahren zum Versehen von Haut, Haaren, Oberflächen oder textilen Fasern mit
(i) einer Maiglöckchen-Geruchsnote
   sowie (ii) gegebenenfalls
(ii)a) einer, mehrerer oder sämtlicher Geruchsnoten ausgewählt aus der Gruppe bestehend aus blumig, fruchtig, grün, wässrig und aldehydig
   und/oder
(ii)b) eines, mehrerer oder sämtlicher Eindrücke ausgewählt aus der Gruppe bestehend aus natürlich, frisch, pflegend, sanft, warm, komplex und strahlend;
umfasst folgende Schritte:
- Bereitstellen
   - einer oder mehrerer Verbindungen der Formel (I) wie oben definiert, oder
   - einer erfindungsgemäßen Mischung wie oben definiert
- Applizieren der Verbindung(en)der Formel (I) bzw. der Mischung auf Haut, Haare, Oberfläche oder textile Fasern.

Hinsichtlich bevorzugter Ausgestaltungen gelten die obigen Ausführungen entsprechend.

### Ausführunasbeispiele

### Beispiel 1: Synthese verschiedener Acetale (Vorprodukte erfindungsgemäß zu verwendender Verbindungen)

### Beschreibung der Synthese am Beispiel von 1-(2,2-Diethoxyethoxy)-3-methylbutan (Formel (IIa). Vorprodukt der Verbindung der Formel (Ia))

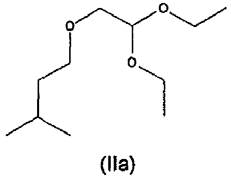

In eine Mischung aus 125 mL Toluol und 13.1 g Natriumhydrid (60%ig in Mineralöl, 329 mmol, 1.20 Äquiv.) werden bei 30-40 °C 24.1 g 3-Methylbutan-1-ol (274 mmol, 1.00 Äquiv.) gelöst in 75 mL Toluol zugetropft. Die Reaktionsmischung wird 10 Minuten zum Sieden erhitzt und 50 mL Toluol werden zugegeben. Die Reaktionsmischung wird 20 Minuten am Rückfluss gekocht. Bei dieser Temperatur werden 54.0 g 2-Bromo-1,1-diethoxyethan (274 mmol, 1.00 Äquiv.) zugetropft und die Lösung anschließend 3.5 Tage gerührt. Nach Abkühlung wird der Ansatz mit 50 mL gesättigte Ammoniumchloridlösung und 50 mL Wasser versetzt. Die wässrige Phase wird dreimal mit 150 mL Essigester extrahiert und die vereinigten organischen Phasen zweimal mit 50 mL gesättigte Natriumchloridlösung gewaschen. Nach Entfernung des Lösungsmittels wird das Rohprodukt (52.0 g, 70%ig, 65% der Theorie) durch eine Destillation gereinigt. Es werden 21 g 1-(2,2-Diethoxyethoxy)-3-methylbutan in 96%iger Reinheit isoliert. Dies entspricht einer Ausbeute von 37% der Theorie.

Siedepunkt: 51 °C / 0.74 mbar. - MS: m/z (%) = 158 (1), 103 (100), 91 (9), 75 (62), 71 (28), 60 (8), 47 (67), 43 (39), 29 (22). - ¹H NMR (400 MHz, CDCl₃): δ (ppm) = 0.90 (d, J = 6.7 Hz, 6 H), 1.22 (t, J = 7.0 Hz, 6 H), 1.47 (q, J = 6.7 Hz, 2 H), 1. 70 (sept., J = 6.7 Hz, 1 H), 3.47 (d, J = 5.3 Hz, 2 H), 3.51 (t, J = 6.8 Hz, 2 H), 3.57 (dq, J = 9.4, 7.0 Hz, 2 H), 3.70 (dq, J = 9.4, 7.1 Hz, 2 H), 4.62 (t, J = 5.3 Hz, 1 H). - ¹³C NMR (100 MHz, CDCl₃): δ (ppm) = 15.4 (2 x CH₃), 22.6 (2 x CH₃), 25.0 (CH), 38.4 (CH₂), 62.2 (2 x CH₂), 70.1 (CH₂), 71.5 (CH₂), 101.2 (CH).

Die nachfolgend beschriebenen Verbindungen wurden analog der oben beschriebenen Synthese von 1-(2,2-Diethoxyethoxy)-3-methylbutan hergestellt.

### 1-(2,2-Diethoxyethoxy)-4-isopropylcyclohexan (Formel (IIb), Vorprodukt der Verbindung der Formel (Ib))

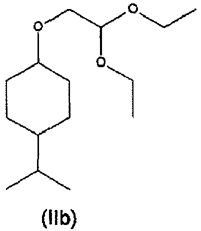

Siedepunkt: 78 °C / 0.15 mbar. - MS: m/z (%) = 125 (6), 103 (100), 88 (8), 75 (35), 69 (15), 47 (29), 29 (7). - ¹H NMR (400 MHz, CDCl₃) für trans-Isomer: δ (ppm) = 0.85 (d, J = 6.8 Hz, 6 H), 0.97-1.02 (m, 3 H), 1.19-1.24 (m, 2 H), 1.22 (t, J = 7.0 Hz, 6 H), 1.37-1.45 (m, 1 H), 1.71-1.76 (m, 2 H), 2.02-2.08 (m, 2 H), 3.20 (tt, J = 10.9, 4.2 Hz, 1 H), 3.50 (d, J = 5.3 Hz, 2 H), 3.57 (dq, J = 9.4, 7.0 Hz, 2 H), 3.70 (dq, J = 9.4, 7.1 Hz, 2 H), 4.60 (t, J = 5.3 Hz, 1 H). - ¹³C NMR (100 MHz, CDCl₃) *trans*-Isomer: δ (ppm) = 15.4 (2 x CH₃), 20.0 (2 x CH₃), 27.9 (2 x CH₂), 32.4 (2 x CH₂), 32.5 (CH), 43.4 (CH), 62.2 (2 x CH₂), 68.8 (CH₂), 79.3 (CH), 101.6 (CH).

### 1-(2,2-Diethoxyethoxy)-3,5,5-trimethylhexan (Formel (IId), Vorprodukt der Verbindung der Formel (Id))

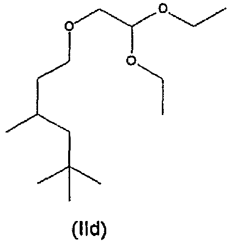

Siedepunkt: 64 °C / 0.27 mbar. - MS: m/z (%) = 125 (3), 103 (100), 88 (10), 75 (32), 69 (9), 57 (26), 47 (26), 41 (8), 29 (10). - ¹H NMR (400 MHz, CDCl₃): δ (ppm) = 0.89 (s, 9 H), 0.92 (d, J = 6.6 Hz, 3 H), 1.05 (dd, J = 14.0, 6.1 Hz, 1 H), 1.22 (dd, J = 13.9, 3.4 Hz, 1 H), 1.22 (t, J = 7.1 Hz, 3 H), 1.22 (t, J = 7.1 Hz, 3 H), 1.37-1.46 (m, 1 H), 1.54-1.62 (m, 2 H), 3.43-3.51 (m, 4 H), 3.57 (dq, J = 9.4, 7.1 Hz, 1 H), 3.58 (dq, J = 9.4, 7.1 Hz, 1 H), 3.70 (dq, J = 9.4, 7.1 Hz, 1 H), 3.71 (dq, J = 9.4, 7.1 Hz, 1 H), 4.62 (t, J = 5.3 Hz, 1 H). - ¹³C NMR (100 MHz, CDCl₃): δ (ppm) = 15.4 (2 x CH₃), 22.8 (CH₃), 26.2 (CH), 30.0 (3 x CH₃), 31.1 (C_{quart.}), 39.0 (CH₂), 51.3 (CH₂), 62.2 (CH₂), 62.3 (CH₂), 70.1 (CH₂), 71.5 (CH₂), 101.2(CH).

### 2-(2,2-Diethoxyethoxy)octan (Formel (IIe), Vorprodukt der Verbindung der Formel (Ie))

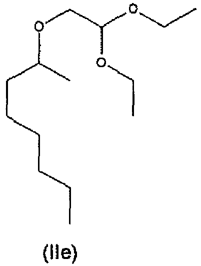

Siedepunkt: 62 °C / 0.18 mbar. - MS: m/z (%) = 103 (100), 89 (14), 75 (43), 57 (13), 47 (38), 29 (14). - ¹H NMR (400 MHz, CDCl₃): δ (ppm) = 0.88 (t, J = 7.0 Hz, 3 H), 1.13 (d, J = 6.2 Hz, 3 H), 1.21 (t, J = 7.0 Hz, 3 H), 1.22 (t, J = 7.0 Hz, 3 H), 1.26-1.40 (m, 8 H), 1.50-1.56 (m, 2 H), 3.40 (dd, J = 10.3, 5.3 Hz, 1 H), 3.41-3.47 (m, 1 H), 3.52 (dd, J = 10.3. 5.1 Hz, 1 H), 3.57 (dq, J = 9.3, 7.0 Hz, 1 H), 3.58 (dq, J = 9.3, 7.0 Hz, 1 H), 3.69 (dq, J = 9.3, 6.9 Hz, 1 H), 3.70 (dq, J = 9.3, 6.9 Hz, 1 H), 4.59 (t, J = 5.3 Hz, 1 H). - ¹³C NMR (100 MHz, CDCl₃): δ (ppm) = 14.1 (CH₃), 15.4 (2 x CH₃), 19.6 (CH₃), 22.6 (CH₂), 25.6 (CH₂), 29.4 (CH₂), 31.9 (CH₂), 36.6 (CH₂), 62.1 (CH₂), 62.2 (CH₂), 69.2 (CH₂), 76.3 (CH), 101.6 (CH).

### Beispiel 2: Synthese von 1-(2,2-Diethoxyethoxy)-4-isopropylbenzen (Formel (IIc), Vorprodukt der Verbindung der Formel (Ic))

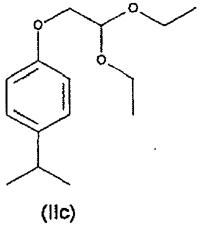

Zu einer Lösung aus 100 mL Dimethylsulfoxid und 12.4 g Kaliumhydroxid (220 mmol, 1.20 Äquiv.) werden 25.0 g 4-Isopropylphenol (184 mmol, 1.00 Äquiv.) gelöst in 100 mL Dimethylsulfoxid und anschließend 36.2 g 2-Bromo-1,1-diethoxyethan (184 mmol, 1.00 Äquiv.) zugetropft. Die Reaktionsmischung wird 4 Stunden am Rückfluss gekocht. Nach Abkühlung wird der Ansatz über Eis geschüttet und 500 mL Wasser werden dazu gegeben. Die wässrige Phase wird viermal mit 100 mL Diethylether extrahiert und die vereinigten organischen Phasen mit 100 mL Natronlauge, viermal mit 100 mL Wasser und 100 mL gesättigte Natriumcarbonatlösung gewaschen. Nach Entfernung des Lösungsmittels erhält man 40.3 g 1-(2,2-Diethoxyethoxy)-4-isopropylbenzen in 97%iger Reinheit. Dies entspricht einer Ausbeute von 84% der Theorie.

Siedepunkt: 107 °C /1.0 mbar. - MS: m/z (%) = 252 (M⁺, 5), 163 (7), 119 (19), 103 (100), 91 (14), 75 (52), 61 (6), 47 (32), 43 (6), 29 (18). - ¹H NMR (400 MHz, CDCl₃): δ (ppm) = 1.22 (d, J = 6.9 Hz, 6 H), 1.24 (t, J = 7.0 Hz, 6 H), 2.85 (sept. J = 6.9 Hz, 1 H), 3.63 (dq, J = 9.4, 7.1 Hz, 2 H), 3.76 (dq, J = 9.4, 7.0 Hz, 2 H), 3.99 (d, J = 5.2 Hz, 2 H), 4.83 (t, J = 5.3 Hz, 1 H), 6.86 (d, J = 8.8 Hz, 2 H), 7.13 (d, J = 8.8 Hz, 2 H). - ¹³C NMR (100 MHz, CDCl₃): δ (ppm) = 15.4 (2 x CH₃), 24.2 (2 x CH₃), 33.3 (CH), 62.4 (2 x CH₂), 68.6 (CH₂), 100.5 (CH), 114.5 (2 x CH), 127.2 (2 x CH), 141.4 (C_{quart.}), 156.7 (C_{quart.}).

### Beispiel 3: Synthese verschiedener Oxyacetaldehyde (erfindungsgemäß zu verwendende Verbindungen)

### Beschreibung derSynthese am Beispiel von 2-(3-Methylbutoxy)acetaldehyd (2-Isopentyloxyacetaldehyd, Formel (Ia))

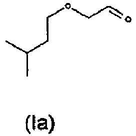

18.0 g 1-(2,2-Diethoxyethoxy)-3-methylbutan (82.8 mmol) werden in 47.0 g Ameisensäure zugetropft und die Reaktionsmischung 2 Stunden gut gerührt. Nach beendeter Reaktion wird die Reaktionslösung mit 100 mL Essigester verdünnt und mit 100 mL Wasser gequencht. Die Phasen werden getrennt. Die organische Phase wird mit 50 mL gesättigter Natriumhydrogencarbonatlösung und 70 g Natriumhydrogencarbonat neutral gewaschen. Die wässrige Phase wird anschließend zweimal mit 50 mL Essigester extrahiert. Nach Entfernung des Lösungsmittels wird das Rohprodukt (11.6 g, 85%ig, 91% der Theorie) durch eine Destillation gereinigt. Es werden 2.5 g 2-(3-Methylbutoxy)acetaldehyd in 99%iger Reinheit isoliert. Dies entspricht einer Ausbeute von 23% der Theorie. Das Produkt wird mit Tocopherol stabilisiert.

Siedepunkt: 72 °C / 40.6 mbar. - MS: m/z (%) = 101 (18), 71 (34), 57 (17), 43 (100), 29 (18). - ¹H NMR (400 MHz, CDCl₃): δ (ppm) = 0.92 (d, J = 6.6 Hz, 6 H), 1.53 (dt, J = 7.0, 6.7 Hz, 2 H), 1.69-1.78 (m, 1 H), 3.56 (t, J = 6.7 Hz, 2 H), 4.06 (d, J = 0.9 Hz, 2 H), 9.74 (t, J = 0.9 Hz, 1 H). - ¹³C NMR (100 MHz, CDCl₃): δ (ppm) = 22.6 (2 x CH₃), 25.0 (CH), 38.3 (CH₂), 70.6 (CH₂), 76.3 (CH₂), 201.2 (CHO).

Die nachfolgend beschriebenen Verbindungen wurden analog der oben beschriebenen Synthese von 2-(3-Methylbutoxy)acetaldehyd hergestellt.

### 2-(4-(Isopropylcyclohexyloxy)acetaldehyd (Formel (Ib))

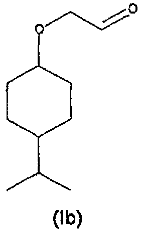

Siedepunkt: 62 °C / 0.31 mbar. - MS: m/z (%) = 125 (62), 85 (36), 69 (100), 54 (30), 41 (32), 29 (8). - ¹H NMR (400 MHz, CDCl₃) für trans-Isomer: δ (ppm) = 0.86 (d, J = 6.9 Hz, 6 H), 0.95-1.04 (m, 3 H), 1.22-1.32 (m, 2 H), 1.38-1.46 (m, 1 H), 1.74-1.81 (m, 2 H), 2.04-2.12 (m, 2 H), 3.25 (tt, J = 10.8, 4.5 Hz, 1 H), 4.10 (d, J = 1.1 Hz, 2 H), 9.74 (t, J = 1.1 Hz, 1 H). - ¹³C NMR (100 MHz, CDCl₃) *trans-*Isomer: δ (ppm) = 19.9 (2 x CH₃), 27.8 (2 x CH₂), 32.2 (2 x CH₂), 32.4 (CH), 43.2 (CH), 73.9 (CH₂), 80.0 (CH), 201.9 (CHO).

### 2-(4-Isopropylphenoxy)acetaldehyd (Formel (Ic))

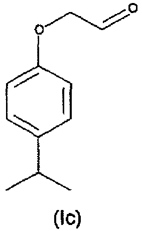

Siedepunkt: 74 °C / 0.41 mbar. - MS: m/z (%) = 178 (35), 163 (100), 121 (17), 119 (18), 107 (13), 105 (25), 103 (15), 91 (46), 77 (25), 41 (12). - ¹H NMR (400 MHz, CDCl₃): δ (ppm) = 1.22 (d, J = 6.9 Hz, 6 H), 2.87 (sept. J = 6.9 Hz, 1 H), 4.54 (d, J = 1.1 Hz, 2 H), 6.83 (d, J = 8.9 Hz, 2 H), 7.17 (d, J = 8.9 Hz, 2 H), 9.86 (t, J = 1.1 Hz, 1 H). - ¹³C NMR (100 MHz, CDCl₃): δ (ppm) = 24.1 (2 x CH₃), 33.3 (CH), 72.9 (CH₂), 114.4 (2 x CH), 127.6 (2 x CH), 142.5 (C_{quart.}), 155.6 (C_{quart.}), 199.8 (CHO).

### 2-(3,5,5-Trimethylhexoxy)acetaldehyd (Formel (Id))

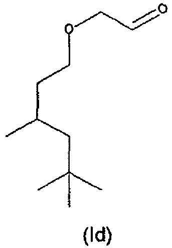

Siedepunkt: 54 °C / 0.33 mbar. - MS: m/z (%) = 125 (5), 111 (7), 101 (22), 83 (7), 69 (41), 57 (100), 41 (25), 29 (12), - ¹H NMR (400 MHz, CDCl₃): δ (ppm) = 0.90 (s, 9 H), 0.94 (d, J = 6.6 Hz, 3 H), 1.08 (dd, J = 14.0, 6.1 Hz, 1 H), 1.23 (dd, J = 14.0, 3.4 Hz, 1 H), 1.44-1.51 (m, 1 H), 1.60-1.69 (m, 2 H), 3.52-3.58 (m, 2 H), 4.05 (d, J = 1.0 Hz, 2 H), 9.75 (t, J = 1.0 Hz, 1 H). - ¹³C NMR (100 MHz, CDCl₃): δ (ppm) = 22.7 (CH₃), 26.1 (CH), 30.0 (3 x CH₃), 31.1 (C_{quart.}), 38.8 (CH₂), 51.2 (CH₂), 70.5 (CH₂), 76.3 (CH₂), 201.3 (CHO).

### 2-[(1-Methylheptyl)oxy]acetaldehyd (Formel (Ie))

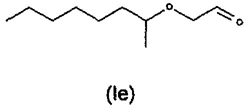

Siedepunkt: 45 °C / 0.29 mbar. - MS: m/z (%) = 113 (29), 71 (85), 57 (100), 43 (66), 29 (23). -¹H NMR (400 MHz, CDCl₃): δ (ppm) = 0.88 (t, J = 6.6 Hz, 3 H), 1.18 (d, J = 6.1 Hz, 3 H), 1.25-1.35 (m, 8 H), 1.38-1.47 (m, 1 H), 1.59-1.65 (m, 1 H), 3.44-3.52 (m, 1 H), 4.01 (dd, J = 17.8, 1.1 Hz, 1 H), 4.08 (dd, J = 17.8, 1.1 Hz, 1 H), 9.74 (t, J = 1.1 Hz, 1 H). ¹³C NMR (100 MHz, CDCl₃): δ (ppm) = 14.1 (CH₃), 19.4 (CH₃), 22.6 (CH₂), 25.4 (CH₂), 29.4 (CH₂), 31.8 (CH₂), 36.3 (CH₂), 74.1 (CH₂), 77.1 (CH), 201.9 (CHO).

### Beispiel 4: Fine Fragrance

| **Bestandteil** | **Parfümöl A1** (Vergleich) | **Parfümöl A2** (erfindungsgemäß) |
|---|---|---|
| ETHYL ACETOACETATE | 4,0 | 4,0 |
| HEXENOL CIS-3 10% DPG | 3,0 | 3,0 |
| HEXENYL ACETATE CIS-3 10% DPG | 3,0 | 3,0 |
| HEXENYL BENZOATE CIS-3 10% DPG | 3,0 | 3,0 |
| VERTOCITRAL 10% DPG | 5,0 | 5,0 |
| CYCLOGALBANAT® 10% DPG | 3,0 | 3,0 |
| STYRALYL ACETATE 10% DPG | 6,0 | 6,0 |
| BERGAMOT OIL BERGAPTEN FREE FF | 6,0 | 6,0 |
| MANDARIN OIL DIST. DECOL | 15,0 | 15,0 |
| METHYL ANTHRANILATE 10% DPG | 5,0 | 5,0 |
| RED BERRY EXTR. | 2,0 | 2,0 |
| DECALACTONE GAMMA | 2,0 | 2,0 |
| ALLYL CAPROATE 10% DPG | 5,0 | 5,0 |
| PRUNELLA TYPE BASE | 6,0 | 6,0 |
| HELIONAL | 20,0 | 20,0 |
| MUGETANOL | 10,0 | 10,0 |
| ETHYL LINALOOL | 35,0 | 35,0 |
| CITRONELLOL 950 | 10,0 | 10,0 |
| GERANIOL SUPER | 3,0 | 3,0 |
| ROSAPHEN® | 8,0 | 8,0 |
| CITRONELLYL ACETATE EXTRA | 2,0 | 2,0 |
| DAMASCONE BETA 10% DPG | 6,0 | 6,0 |
| BENZYL ACETATE | 10,0 | 10,0 |
| HEDION HC/30 | 30,0 | 30,0 |
| HEDIONE | 140,0 | 140,0 |
| HEXYL CINNAMIC ALDEHYDE ALPHA | 30,0 | 30,0 |
| LACTOJASMONE | 3,0 | 3,0 |
| BENZYL SALICYLATE | 80,0 | 80,0 |
| HEXENYL SALICYLATE CIS-3 | 30,0 | 30,0 |
| METHYL IONONE GAMMA COEUR | 3,0 | 3,0 |
| CLOVE BUD OIL | 1,0 | 1,0 |
| VANILLIN | 1,5 | 1,5 |
| COUMARIN | 1,0 | 1,0 |
| AMBERWOOD® F | 5,0 | 5,0 |
| CASHMERAN | 8,0 | 8,0 |
| CEDRENE | 15,0 | 15,0 |
| ISO E SUPER NON DISCOLORING | 50,0 | 50,0 |
| BRAHMANOL® | 25,0 | 25,0 |
| SANDALORE | 5,0 | 5,0 |
| AMBROXIDE | 2,0 | 2,0 |
| AMBRETTOLIDE | 5,0 | 5,0 |
| AURELIONE® | 16,0 | 16,0 |
| GLOBALIDE® | 24,0 | 24,0 |
| MACROLIDE® SUPRA | 16,0 | 16,0 |
| INDOLE FF 10% DPG | 5,0 | 5,0 |
| **2-(3-Methylbutoxy)acetaldehyd (Formel (Ia))** | | **50,0** |
| DIPROPYLENE GLYCOL | 332,5 | 282,5 |
| | 1.000,0 | 1.000,0 |

Durch den Zusatz von 2-(3-Methylbutoxy)acetaldehyd (Formel (Ia)) wird bei einer Dosierung von 6% des Parfümöls A2 in Ethanol die blumige Note verstärkt Weiterhin verleiht der Zusatz von 2-(3-Methylbutoxy)acetaldehyd der Komposition mehr Natürlichkeit.

### Beispiel 5: Bodylotion

| **Komponente** | **Ges.-%** | **Gew.-%** |
|---|---|---|
| Paraffinöl | 5,00 | 5,00 |
| Isopropylpalmitat | 5,00 | 5,00 |
| Cetylalkohol | 2,00 | 2,00 |
| Bienenwachs | 2,00 | 2,00 |
| Ceteareth-20 | 2,00 | 2,00 |
| PEG-20-Glycerylstearat | 1,50 | 1,50 |
| Glycerin | 3,00 | 3,00 |
| Phenoxyethanol | 0,50 | - |
| Parabene (Mischung aus Methyl-, Ethyl-, Propyl-, Butyl-, Isobutylparaben) | - | 0,50 |
| Parfümöl B1 bzw. B2 | 0,50 | 0,50 |
| Wasser | Ad 100 | Ad 100 |

### Parfümöle für diese Bodylotion:

| **Bestandteil** | **Parfümöl B1** (Vergleich) | **Parfümöl B2** (erfindungsgemäß) |
|---|---|---|
| NONADIENAL TRANS,CIS-2,6 5% TEC 20% DPG | 2,0 | 2,0 |
| ETHYL ACETOACETATE | 3,0 | 3,0 |
| FARENAL® 10% DPG | 5,0 | 5,0 |
| VERTOCITRAL | 3,0 | 3,0 |
| CYCLOGALBANAT® 10% DPG | 2,0 | 2,0 |
| STYRALYL ACETATE | 3,0 | 3,0 |
| MELONAL® | 0,5 | 0,5 |
| DIHYDRO MYRCENOL | 15,0 | 15,0 |
| LINALYL ACETATE | 20,0 | 20,0 |
| LEMON OIL TERPENES FLAVOR WONF | 8,0 | 8,0 |
| EUCALYPTOL NAT. 10% DPG | 0,5 | 0,5 |
| HEXYL ACETATE | 1,5 | 1,5 |
| ISOAMYL ACETATE 10% DPG | 4,0 | 4,0 |
| PRENYL ACETATE 10% DPG | 4,0 | 4,0 |
| ALDEHYDE C14 SO-CALLED | 2,0 | 2,0 |
| ETHYL METHYL BUTYRATE-2 | 1,0 | 1,0 |
| ALLYL CYCLOHEXYL PROPIONATE | 2.0 | 2,0 |
| ALDEHYDE C16 SO-CALLED | 1,0 | 1,0 |
| FRAGOLANE® | 0,5 | 0,5 |
| MAJANTOL® | 25,0 | 25,0 |
| LlNALOOL | 40,0 | 40,0 |
| DIMETHYL BENZYL CARBINOL | 10,0 | 10,0 |
| TERPINEOL PURE | 10,0 | 10,0 |
| PHENIRAT® | 30,0 | 30,0 |
| CITRONELLOL 950 | 15,0 | 15,0 |
| GERANIOL 60 | 10,0 | 10,0 |
| CITRONELLYL ACETATE EXTRA | 2,0 | 2,0 |
| HEDIONE | 90,0 | 90,0 |
| HEXYL CINNAMIC ALDEHYDE ALPHA | 35,0 | 35,0 |
| HEXYL SALICYLATE | 160,0 | 160,0 |
| METHYL OCTIN CARBONATE 10% DPG | 2,0 | 2,0 |
| CALONE 1951 10% DPG | 1,0 | 1,0 |
| GALAXOLIDE 50% IN IPM | 20,0 | 20,0 |
| ANETHOL SUPRA 21,5 CELSIUS | 2,0 | 2,0 |
| AGRUMEX HC | 15,0 | 15,0 |
| ORYCLON SPECIAL | 40,0 | 40,0 |
| **2-(4-Isopropytphenoxy)acetaldehyd (Formel (Ic))** | | **15,0** |
| DIPROPYLENE GLYCOL | 415,0 | 400,0 |
| | 1.000,0 | 1.000,0 |

Durch den Zusatz von 2-(4-Isopropylphenoxy)acetaldehyd (Formel (Ic)) zu dem Parfümöl B2 ergibt sich eine frischere blumige Note des Duschgels. Außerdem wird die Topnote wässriger und der Nachgeruch pflegender.

### Beispiel 6: Weichspüler

| **Material** | **Hersteller** | **Chemischer Name** | **Funktion** | **Gew.-%** |
|---|---|---|---|---|
| Entionisiertes Wasser | | Wasser | Lösungsmittel | 72,4 |
| Rewoquat WE 18 | Evonic Goldschmidt GmbH | Dialkylesterammoniumethosulfat | Kationisches Tensid | 16,6 |
| Mergal K9N | Honeywell Austria GmbH | 5-Chloro-2-methyl-3-(2H)-isothiazolon und 2-methyl-3-(2H)-isothiazolon | Konservie-rungsmittel | 0,10 |
| Dow Corning 1520 Antifoam | Dow Corning GmbH, Deutschland | Polydimethyl-siloxane | Entschäumer | 0,30 |
| Magnesiumchlorid 1%ige Lösung | | MagnesiumchloridLösung | Konsistenzgeber | 10,00 |
| Parfumöl C1 bzw. C2 | Symrise | | Parfüm (Fragrance) | 0,60 |

### Parfümöle für diesen Weichspüler:

| **Bestandteil** | **Parfümöl C1** (Vergleich) | **Parfümöl C2** (erfindungsgemäß) |
|---|---|---|
| ALDEHYDE C10 | 0,5 | 0,5 |
| ALDEHYDE C11 ISO | 2,0 | 2,0 |
| ALDEHYDE C11 UNDECYLENIC | 12,0 | 12,0 |
| ALDEHYDE C12 MNA | 6,0 | 6,0 |
| FARENAL® | 1,0 | 1,0 |
| VERTOCITRAL | 8,0 | 8,0 |
| ALLYL AMYL GLYCOLATE | 1,0 | 1,0 |
| STYRALYL ACETATE | 1,5 | 1,5 |
| DIHYDRO MYRCENOL | 65,0 | 65,0 |
| AGRUNITRIL | 1,0 | 1,0 |
| PEONILE | 15,0 | 15,0 |
| METHYL ANTHRANILATE | 10,0 | 10,0 |
| NEROLIONE 10% DPG | 1,5 | 1,5 |
| ROSEMARY OIL BM | 7,0 | 7,0 |
| SAGE OFFICINALE OIL DALM. | 3,0 | 3,0 |
| ISOBORNYL ACETATE | 40,0 | 40,0 |
| PRENYL ACETATE | 0,5 | 0,5 |
| ISOAMYL BUTYRATE | 0,5 | 0,5 |
| ALDEHYDE C14 SO-CALLED | 5,0 | 5,0 |
| MANZANATE | 0,5 | 0,5 |
| MUGETANOL | 5,0 | 5,0 |
| DIMETHYL BENZYL CARBINYL ACETATE | 2,5 | 2,5 |
| ROSE OXIDE D | 3,0 | 3,0 |
| ANTHER | 0,5 | 0,5 |
| PHENYLETHYL ALCOHOL | 35,0 | 35,0 |
| CITRONELLOL 950 | 15,0 | 15,0 |
| GERANIOL 60 | 10,0 | 10,0 |
| ISODAMASCON® | 2,0 | 2,0 |
| ROSACETATE | 35,0 | 35,0 |
| CRESYL METHYL ETHER PARA | 0,5 | 0,5 |
| BENZYL ACETATE | 20,0 | 20,0 |
| HEXYL CINNAMIC ALDEHYDE ALPHA | 40,0 | 40,0 |
| METHYL BENZOATE | 1,0 | 1,0 |
| AMYL SALICYLATE N/ISO | 10,0 | 10,0 |
| BENZYL SALICYLATE | 70,0 | 70,0 |
| MYSORE ACETATE | 15,0 | 15,0 |
| PARMANYL® | 0,5 | 0,5 |
| EUGENOL | 10,0 | 10,0 |
| ETHYL VANILLIN | 0,5 | 0,5 |
| COUMARIN | 3,0 | 3,0 |
| AGRUMEX HC | 45,0 | 45,0 |
| HERBAFLORAT | 25,0 | 25,0 |
| AMBERWOOD® F | 5,0 | 5,0 |
| ISO E SUPER | 170,0 | 170,0 |
| PATCHOULI OIL DECOL | 2,5 | 2,5 |
| SANDRANOL® | 10,0 | 10,0 |
| ISOBUTYL QUINOLlNE | 1,0 | 1,0 |
| EVERNYL | 0,5 | 0,5 |
| AMBROXIDE | 1,0 | 1,0 |
| GALAXOLIDE 50% IN IPM | 120,0 | 120,0 |
| INDOFLOR® CRYST. | 0,5 | 0,5 |
| **2-[(1-Methylheptyl)oxy]acetaldehyd (Formel (Ie))** | | **60,0** |
| DIPROPYLENE GLYCOL | 220,0 | 160,0 |
| | 1.000,0 | 1.000,0 |

Durch den Zusatz von 2-[(1-Methylheptyl)oxy]acetaldehyd (Formel (Ie)) zu dem Parfümöl C2 ergibt sich eine natürlichere, runde aldehydische Note des Weichspülers.

### Beispiel 7: Seife

| **Material** | **Hersteller** | **Chemischer Name** | **Funktion** | **Gew.-%** |
|---|---|---|---|---|
| Entionisiertes Wasser | | Water | Lösungsmittel | 2,0 |
| Seifen Basen Mix | Verschiedene | Sodium tallowates / palmitates | Tenside | 95,8 |
| Titan Dioxid | Kronos Titan GmbH Deutschland | Titanium Dioxide | Farbstoff / Aufheller | 1,0 |
| Parfumöl D1 bzw. D2 | Symrise | | Parfum (Fragrance) | 1,2 |

### Parfümöle für diese Seife:

| **Bestandteil** | **Parfümöl D1** (Vergleich) | **Parfümöl D2** (erfindungsgemäß) |
|---|---|---|
| ALDEHYDE C8 | 10,0 | 10,0 |
| ALDEHYDE C10 | 14,0 | 14,0 |
| ALDEHYDE C11 UNDECYLIC | 5,0 | 5,0 |
| ALDEHYDE C12 MNA | 4,0 | 4,0 |
| ISOAMYL ALCOHOL | 6,0 | 6,0 |
| VERTOCITRAL | 4,0 | 4,0 |
| ALLYL AMYL GLYCOLATE | 4,0 | 4,0 |
| DIHYDRO MYRCENOL | 120,0 | 120,0 |
| CITRAL 95 | 30,0 | 30,0 |
| AGRUNITRIL | 80,0 | 80,0 |
| CITRONITRILE | 40,0 | 40,0 |
| CITRYLAL | 4,0 | 4,0 |
| ORANGE OIL TERPENES | 250,0 | 250,0 |
| METHYL ANTHRANILATE | 1,5 | 1,5 |
| TERPINEOL HEAD FRACTION | 10,0 | 10,0 |
| EUCALYPTOL NAT. | 10,0 | 10,0 |
| THYMOL CRYST | 6,0 | 6,0 |
| BORNYL ACETATE L CRYST. | 82,5 | 82,5 |
| CAMPHOR DL | 20,0 | 20,0 |
| JASMAPRUNAT | 5,0 | 5,0 |
| ALDEHYDE C14 SO-CALLED | 2,0 | 2,0 |
| MANZANATE | 0,5 | 0,5 |
| ALLYL HEPTOATE | 2,5 | 2,5 |
| ROSE OXIDE L | 0,5 | 0,5 |
| DAMASCONE ALPHA | 0,5 | 0,5 |
| HEDIONE | 10,0 | 10,0 |
| AMYL SALICYLATE N/ISO | 60,0 | 60,0 |
| HEXYL SALICYLATE | 35,0 | 35,0 |
| COUMARIN | 2,5 | 2,5 |
| AGRUMEX HC | 30,0 | 30,0 |
| ORYCLON SPECIAL | 55,0 | 55,0 |
| PATCHOULI OIL DECOL. | 2,0 | 2,0 |
| SANDRANOL® | 1,5 | 1,5 |
| TONALIDE | 2,0 | 2,0 |
| **2-(3,5,5-Trimethylhexoxy)acetaldehyd (Formel (Id))** | | **40,0** |
| DIPROPYLENE GLYCOL | 130,0 | 90,0 |
| | 1.000,0 | 1.000,0 |

Der Zusatz von 2-(3,5,5-Trimethylhexoxy)acetaldehyd (Formel (Id)) zu dem Parfümöl D2 ergibt eine natürlichere blumige Note der Seife. Weiterhin verleiht 2-(3,5,5-Trimethylhexoxy)acetaldehyd dem Duft der Seife mehr Kraft und Fülle.

### Beispiel 8: Waschpulver

| **Material** | **Hersteller** | **Chemischer Name** | **Funktion** | **Gew.-%** |
|---|---|---|---|---|
| Natrium Metasilicat Pentahydrat | Akzo Nobel Chemicals, Deutschland | Sodium Metasilicate Pentahydrate | | 48,0 |
| Natriumhydrogencarbonat | Verschiedene | Sodium hydrogen carbonate | Alkali | 15,0 |
| Natriumpercarbonat | Verschiedene | Sodium carbonate peroxyhydrate | Bleichmittel | 15,0 |
| Peractive AC Blue | Clariant GmbH, Deutschland | TAED/Na-Carboxymethylcellulose | Aktivator | 5,00 |
| Genapol OA-080 | Clariant GmbH, Deutschland | Oxoalkohol C 14-15, 8EO | Nichtionisches Tensid | 3,00 |
| Texapon K12 Pulver | Cognis Deutschland GmbH | Sodium Lauryl Sulphate C12 | Anionisches Tensid | 7,00 |
| Tinopal CBS-X | Ciba, Deutschland | | Aufheller | 0,50 |
| Savinase 6.0 T, Type W | Novozymes | Protease | Enzym | 0,40 |
| Termamyl 120 T | Novozymes | Alpha-Amylase | Enzym | 0,30 |
| Natriumsulfat | Verschiende | Sodium Sulphate | Füllstoff | 5,50 |
| Parfumöl E1 bzw. E2 | Symrise | | Parfum (Fragrance) | 0,30 |

### Parfümöle für dieses Waschpulver:

| **Bestandteil** | **Parfümöl E1** (Vergleich) | **Parfümöl E2** (erfindungsgemäß) |
|---|---|---|
| ALDEHYDE 8 | 0,5 | 0,5 |
| HEXENOL CIS-3 | 3,5 | 3,5 |
| VERTOCITRAL | 15,0 | 15,0 |
| STYRALYL ACETATE | 3,0 | 3,0 |
| MELONAL® | 2,0 | 2,0 |
| DIHYDRO MYRCENOL | 60,0 | 60,0 |
| CITRAL 95 | 6,0 | 6,0 |
| ORANGE OIL TERPENES | 20,0 | 20,0 |
| EUCALYPTUS OIL GLOBULUS 80/85% | 10,0 | 10,0 |
| MENTHONE L/ISOMENTHONE D 82/18 | 1,5 | 1,5 |
| HEXYL ACETATE 10% DPG | 5,0 | 5,0 |
| ISOPENTYRATE | 4,0 | 4,0 |
| ETHYL CAPROATE | 1,0 | 1,0 |
| ETHYL METHYL BUTYRATE-2 | 6,0 | 6,0 |
| LINALOOL | 10,0 | 10,0 |
| DIMETHYL BENZYL CARBINYL BUTYRAT | 15,0 | 15,0 |
| PHENYLETHYL ALCOHOL | 15,0 | 15,0 |
| CITRONELLOL 950 | 20,0 | 20,0 |
| GERANIOL 60 | 20,0 | 20,0 |
| ISODAMASCON® | 10,0 | 10,0 |
| CRESYL METHYL ETHER PARA | 1,0 | 1,0 |
| BENZYL ACETONE | 50,0 | 50,0 |
| HEXYL CINNAMIC ALDEHYDE ALPHA | 60,0 | 60,0 |
| DIHYDRO JASMONE | 1,0 | 1,0 |
| METHYL BENZOATE | 0,5 | 0,5 |
| HEXYL SALICYLATE | 10,0 | 10,0 |
| LEGUMINAL | 6,0 | 6,0 |
| IONONE BETA | 20,0 | 20,0 |
| ISORALDEINE 70 | 45,0 | 45,0 |
| EUGENOL | 2,0 | 2,0 |
| ACETOPHENONE 1% DPG | 5,0 | 5,0 |
| AGRUMEX HC | 60,0 | 60,0 |
| ORYCLON SPECIAL | 40,0 | 40,0 |
| HERBAFLORAT | 10,0 | 10,0 |
| HERBYL PROPIONATE | 20,0 | 20,0 |
| KOAVONE | 20,0 | 20,0 |
| VERTOFIX | 40,0 | 40,0 |
| ISO E SUPER | 150,0 | 150,0 |
| SANDRANOL® | 20,0 | 20,0 |
| AMBROXIDE | 2,0 | 2,0 |
| **2-(3-Methylbutoxy)acetaldehyd (Formel (Ia))** | | **60,0** |
| DIPROPYLENE GLYCOL | 270,0 | 210,0 |
| | 1.000,0 | 1.000,0 |

Bei einer Dosierung von 0.3 Gew.-% der Parfümöl E1 bzw. E2 in einem Waschpulver ergibt sich folgender Befund: der Anteil von 2-(3-Methylbutoxy)acetaldehyd (Formel (Ia)) in Parfümöl E2 bewirkt eine Verstärkung der grünen Noten. Weiterhin strahlt die Komposition mit 2-(3-Methylbutoxy)acetaldehyd insgesamt mehr Natürlichkeit aus.

### Beispiel 9: Allzweckreiniger

| **Material** | **Hersteller** | **Chemischer Name** | **Funktion** | **Gew.-%** |
|---|---|---|---|---|
| Entionisiertes Wasser | | Water | Lösungsmittel | 59,6 |
| Mergal K9N | Troy Chemie, Seelze | 5-Chloro-2-methyl-3-(2H)-isothiazolone und 2-methyl-3-(2H)-isothiazolone | Konservierungsmittel | 0,1 |
| Trinatriumcitrat Dihydrat | Verschiedene | Tri Sodium Citrate Dihydrate | Komplexbildner | 3,0 |
| Zetesol NL-2 | Zschimmer & Schwarz, Deutschland | Fatty alcohol C12-14-sulfate, Sodium | Anionisches Tensid | 30,0 |
| Imbentin C/125/055 | Dr. W. Kolb AG Chem. | Fatty alcohol C12-C15, 8EO | Nichtionisches Tensid | 5,0 |
| Ethanol 96% | Verschiedene | Ethanol | Lösungsmittel | 2,0 |
| Parfumöl F1 bzw. F2 | Symrise | | Parfum (Fragrance) | 0,3 |

### Parfümöle für diesen Allzweckreiniger:

| **Bestandteil** | **Parfümöl F1** (Vergleich) | **Parfümöl F2** (erfindungsgemäß) |
|---|---|---|
| ALDEHYDE C10 | 0,5 | 0,5 |
| ALCOHOL C10 | 6,0 | 6,0 |
| HEXENOL CIS-3 | 1,0 | 1,0 |
| LIMONENAL | 4,0 | 4,0 |
| VERTOCITRAL | 8,0 | 8,0 |
| PHENYLACETALDEHYDE 50% DPG | 1,0 | 1,0 |
| MINTONAT | 15,5 | 15,5 |
| METHYL ANTHRANILATE | 1,0 | 1,0 |
| HEXYL ACETATE | 20,0 | 20,0 |
| ALLYL HEPTOATE | 1,0 | 1,0 |
| LINALOOL | 30,0 | 30,0 |
| DIMETHYL BENZYL CARBINYLACETATE | 2,0 | 2,0 |
| PHENYLETHYL ALCOHOL | 150,0 | 150,0 |
| CITRONELLOL 950 | 130,0 | 130,0 |
| GERANIOL SUPER | 35,0 | 35,0 |
| GERANYL ACETATE PURE | 10,0 | 10,0 |
| BENZYL ACETATE | 55,0 | 55,0 |
| HEDIONE | 15,0 | 15,0 |
| HEXYL CINNAMIC ALDEHYDE ALPHA | 130,0 | 130,0 |
| CINNAMIC ALCOHOL | 6,0 | 6,0 |
| ACETOPHENONE 10% DPG | 1,0 | 1,0 |
| JACINTHAFLOR® | 4,0 | 4,0 |
| ISO E SUPER | 20,0 | 20,0 |
| GALAXOLIDE 50% IN IPM | 35,0 | 35,0 |

| | | |
|---|---|---|
| INDOFLOR® CRYST. | 1,0 | 1,0 |
| BHT IONOL | 8,0 | 8,0 |
| **2-(4-Isopropylcyclohexyloxy)acetaldehyd (Formel (Ib)** | | **60,0** |
| DIPROPYLENE GLYCOL | 370,0 | 310,0 |
| | 1.000,0 | 1.000,0 |

Bei einer Dosierung von 0.3% des Parfümöls im Allzweckreiniger ergibt sich folgender Befund: der Anteil von 2-(4-Isopropylcyclohexyloxy)acetaldehyd (Formel (Ib)) in Parfümöl F2 bewirkt eine Verstärkung der grünen Note des Allzweckreinigers. Ferner erscheint die Komposition des Allzweckreiniges mit dem Parfümöl F2 viel sanfter und wärmer als die des Allzweckreinigers mit dem Parfümöl F1.

### Beispiel 10: Shampoo

| **Material** | **Hersteller** | **INCI-Name** | **Gew.-%** |
|---|---|---|---|
| Entionisiertes Wasser | | Water | 71,5 |
| Plantacare PS 10 | Cognis Deutschland GmbH | Sodium Laureth Sulfate, Lauryl Glucoside | 20,0 |
| Euperlan PK 771 | Cognis Deutschland GmbH | Glycol Distearate, Sodium Lauryl Sulfate, Cocamide MEA, Laureth-10 | 6,0 |
| Dragocid Liquid | Symrise | Phenoxyethanol, Methylparaben, Ethylparaben, Butylparaben, Propylparaben, Isobutylparaben | 0,5 |
| Natrium Chlorid | | Sodium Chloride | 1,4 |
| Citronensäure Monohydrat kristallin | | Citric Acid | 0,1 |
| Parfumöl G1 bzw. G2 | Symrise | Parfum (Fragrance) | 0,5 |

### Parfümöle für dieses Perlglanz-Shampoo:

| **Bestandteil** | **Parfümöl G1** (Vergleich) | **Parfümöl G2** (erfindungsgemäß) |
|---|---|---|
| FARENAL® | 3,0 | 3,0 |
| FLORAZON | 0,5 | 0,5 |
| HEXENYL ACETATE CIS-3 | 3,0 | 3,0 |
| VERTOCITRAL | 1,0 | 1,0 |
| DYNASCONE 10% DPG | 2,0 | 2,0 |
| CYCLOGALBANAT® | 2,0 | 2,0 |
| STYRALYL ACETATE | 1,5 | 1,5 |
| DIHYDRO MYRCENOL | 6,0 | 6,0 |
| OXANTHIA 50% IN TEC 10% DPG | 10,0 | 10,0 |
| LEMON OIL ITAL. | 10,0 | 10,0 |
| ORANGE OIL BRASIL | 50,0 | 50,0 |
| HEXYL ACETATE | 2,0 | 2,0 |
| ISOAMYL ACETATE | 0,5 | 0,5 |
| PRENYL ACETATE | 0,5 | 0,5 |
| ETHYL BUTYRATE 10% DPG | 2,0 | 2,0 |
| ALDEHYDE C14 SO-CALLED | 25,0 | 25,0 |
| DECALACTONE GAMMA | 5,0 | 5,0 |
| ETHYL METHYL BUTYRATE-2 | 1,0 | 1,0 |
| ALLYL CAPROATE | 1,5 | 1,5 |
| ALLYL CYCLOHEXYL PROPIONATE | 3,0 | 3,0 |
| ALLYL HEPTOATE | 2,5 | 2,5 |
| MELOZONE | 2,0 | 2,0 |
| CALONE 1951 | 0,5 | 0,5 |
| MUGETANOL | 10,0 | 10,0 |
| LINALOOL | 25,0 | 25,0 |
| DIMETHYL BENZYL CARBINYL ACETATE | 6,0 | 6,0 |
| DIMETHYL BENZYL CARBINYL BUTYRAT | 4,0 | 4,0 |
| PHENYLETHYL ACETATE | 1,5 | 1,5 |
| PHENYLETHYL ALCOHOL | 15,0 | 15,0 |
| CITRONELLOL 950 | 10,0 | 10,0 |
| GERANIOL SUPER | 5,0 | 5,0 |
| GERANYL ACETATE PURE | 15,0 | 15,0 |
| ISODAMASCON® | 2,0 | 2,0 |
| BENZYL ACETATE | 15,0 | 15,0 |
| HEDIONE | 90,0 | 90,0 |
| HEXYL CINNAMIC ALDEHYDE ALPHA | 35,0 | 35,0 |
| JASMONE CIS | 0,5 | 0,5 |
| BENZYL SALICYLATE | 80,0 | 80,0 |
| HEXENYL SALICYLATE CIS-3 | 30,0 | 30,0 |
| ISORALDEINE 70 | 35,0 | 35,0 |
| HERBAFLORAT | 15,0 | 15,0 |
| ISO E SUPER | 15,0 | 15,0 |
| ISOBORNYL CYCLOHEXANOL | 30,0 | 30,0 |
| BRAHMANOL® | 10,0 | 10,0 |
| AMBROXIDE | 1,5 | 1,5 |
| GLOBALIDE® | 5,0 | 5,0 |
| GALAXOLIDE 50% IN DPG | 120,0 | 120,0 |
| **2-(4-Isopropylphenoxy)acetaldehyd (Formel (Ic))** | | **65,0** |
| DIPROPYLENE GLYCOL | 290,0 | 225,0 |
| | 1.000,0 | 1.000,0 |

Bei einer Dosierung von 0.5% des Parfümöls im Shampoo ergibt sich folgender Befund: der Zusatz 2-(4-Isopropylphenoxy)acetaldehyd (Formel (Ic)) in Parfümöl G2 verstärkt den wässrigen-fruchtigen Charakter des Shampoos. Zudem ist in dem Shampoo mit dem Parfümöl G2 die Diffusivität erhöht gegenüber dem Shampoo mit der Parfümöl G1.

### Beispiel 11: Duschgel

| **Material** | **Hersteller** | **INCI-Name** | **Gew.-%** |
|---|---|---|---|
| Entionisiertes Wasser | | Wasser | 76,3 |
| Plantacare PS 10 | Cognis Deutschland GmbH | Sodium Laureth Sulfate, Lauryl Glucoside | 20,0 |
| Dragocid Liquid | Symrise | Phenoxyethanol, Methylparaben, Ethylparaben, Butylparaben, Propylparaben, Isobutylparaben | 0,5 |
| Natrium Chlorid | | Sodium Chloride | 1,4 |
| Citronensäure Monohydrat kristallin | | Citric Acid | 1,3 |
| Parfumöl C1 bzw. C2 aus Beispiel 6 | Symrise | Parfum (Fragrance) | 0,5 |

Bei einer Dosierung von 0.5% des Parfümöls C1 bzw. C2 im Duschgel ergibt sich folgender Befund: der Zusatz von 2-[1-Methylheptyl)oxy]acetaldehyd in Parfümöl C2 verleiht dem Duschgel eine frischere und kosmetischere Note.

### Beispiel 12: Transparente Deo-Stifte (Formulierung A, B) bzw. Deo-Creme-Stifte (Formulierung C, D)

| **Komponente** | **A** | **B** | **C** | **D** |
|---|---|---|---|---|
| | **Gew.-%** | **Gew.-%** | **Gew.-%** | **Gew.-%** |
| Aluminium Zirconium Tetrachlorohydrat - Glycin Komplex | 25,00 | 20,00 | 25,00 | 20,00 |
| Dimethicon (10 Cst) | - | - | 5,00 | 5,00 |
| Cyclopentasiloxan | - | 0,50 | 1,00 | 0,50 |
| Petrolatum | 5,00 | 4,70 | 5,00 | 5,00 |
| Ozokerit | 1,00 | 1,50 | - | - |
| Stearylalkohol | 12,00 | 12,00 | - | - |
| 2-Butyloctansäure | 0,50 | - | 0,50 | - |
| Wachs | - | - | 1,25 | 1,25 |
| PPG-14 Butylether | 9,00 | 9,00 | - | - |
| Gehärtetes Rapsöl | - | - | 5,00 | 5,00 |
| Siliciumdioxid | - | - | 1,00 | - |
| Farnesol | 0,25 | - | 0,25 | - |
| Paraffinöl | 0,50 | 0,50 | - | - |
| Hydriertes Rizinusöl (castor wax) | 3,50 | 3,50 | - | - |
| Talk | 4,00 | 4,00 | - | - |
| Behenylalkohol | 0,20 | 0,20 | - | - |
| d-Panthenyltriacetat | 1,00 | 1,00 | - | - |
| Konservierungsmittel | q.s. | q.s. | q.s. | q.s. |
| Parfümöl H1 bzw. H2 | 1,50 | - | 1,15 | - |
| Parfümöl G2 aus Beispiel 10 | | 0,90 | - | 0,75 |
| Wasser | Ad 100 | Ad 100 | Ad 100 | Ad 100 |

### Parfümöle für die Transparente Deo-Stifte bzw. Deo-Creme-Stifte :

| **Bestandteil** | **Parfümöl H1** (Vergleich) | **Parfümöl H2** (erfindungsgemäß) |
|---|---|---|
| ALDEHYDE C10 | 0,5 | 0,5 |
| ALDEHYDE C12 MNA 10% DPG | 2,0 | 2,0 |
| MINTONAT | 40,0 | 40,0 |
| MELOZONE | 0,5 | 0,5 |
| VERTOCITRAL | 6,0 | 6,0 |
| DIHYDRO MYRCENOL | 120,0 | 120,0 |
| TETRAHYDRO MYRCENOL | 10,0 | 10,0 |
| AGRUNITRIL | 1,0 | 1,0 |
| ORANGE OIL BRASIL | 10,0 | 10,0 |
| TAMARINE TYPE BASE | 10,0 | 10,0 |
| HEXYL ACETATE | 6,0 | 6,0 |
| ALDEHYDE C14 SO-CALLED | 1,0 | 1,0 |
| TETRAHYDRO LINALOOL | 40,0 | 40,0 |
| DIMETHYL BENZYL CARBINYL BUTYRAT | 5,0 | 5,0 |
| ORANGE FLOWER ETHER | 5,0 | 5,0 |
| PHENYLETHYL ACETATE | 6,5 | 6,5 |
| PHENYLETHYL ALCOHOL | 15,0 | 15,0 |
| GERANIOL SUPER | 30,0 | 30,0 |
| ROSAPHEN® | 25,0 | 25,0 |
| ISODAMASCON® | 0,5 | 0,5 |
| HEDIONE | 300,0 | 300,0 |
| UNDECAVERTOL | 0,5 | 0,5 |
| ALLYL IONONE | 2,5 | 2,5 |
| IONONE BETA | 10,0 | 10,0 |
| EUGENOL | 4,0 | 4,0 |
| ISO E SUPER | 30,0 | 30,0 |
| BRAHMANOL® | 2,0 | 2,0 |
| AMBROXIDE | 2,0 | 2,0 |
| GLOBALIDE® | 12,0 | 12,0 |
| GLOBANONE® | 8,0 | 8,0 |
| MACROLIDE® SUPRA | 45,0 | 45,0 |
| **2-(4-Isopropylcyclohexyloxy)acetaldehyd (Formel (Ib))** | | **50,0** |
| DIPROPYLENE GLYCOL | 300,0 | 250,0 |
| | 1.000,0 | 1.000,0 |

### Beispiel 13: Antiperspirant-Roll-On

| **Komponente** | **Gew.-%** | **Gew.-%** |
|---|---|---|
| Caprylyl Trimethicon (SilCare TM Silicone 31 M 50) | 0,30 | 0,30 |
| Steareth-20 (GENAPOL TM HS 200) | 3,00 | 3,00 |
| Steareth-2 (GENAPOL TM HS 020) | 1,50 | 1,50 |
| Dicaprylyl Ether (Cetiol TM OE) | 2,00 | 2,00 |
| Coco Caprylat/Caprat (Cetiol TM LC) | 2,00 | 2,00 |
| Glycerin | 2,00 | 2,00 |
| Glyceryl Stearat (Cutina TM GMS) | 2,00 | 2,00 |
| Octyldodecanol (Eutanol TM G) | 1,00 | 1,00 |
| Stearyl alkohol | 2,50 | 2,50 |
| Aluminiumchlorohydrat gemäß Beispiel 1 der EP 1321431 | 10,00 | 10,00 |
| Avocado Extract Persea Gratissima | 0,30 | 0,20 |
| Parfümöl G2 aus Beispiel 10 | 0,50 | - |
| Parfümöl H2 aus Beispiel 12 | - | 0,60 |
| Wasser | Ad 100 | Ad 100 |

### Beispiel 14: Antiperspirant-Stick

| **Komponente** | **Gew.-%** | **Gew.-%** |
|---|---|---|
| Phenyl Trimethicon (SilCare TM Silicone 15 M 50) | 13,50 | 13,50 |
| Cetearyl Alkohol | Ad 100 | Ad 100 |
| Cetiol CC (Dicaprylyl Carbonat) | 13,50 | 13,50 |
| Stearinsäure | 3,50 | 3,60 |
| PEG-40-Hydriertes Rizinusöl (Emulsogen TM HCO 040) | 4,10 | 4,10 |
| PEG-8 Distearate (Cithrol 4 DS) | 4,10 | 4,10 |
| Petrolatum | 6,90 | 6,90 |
| Aluminiumchlorohydrat | 13,80 | 13,80 |
| Aluminium Zirconium Trichlorohydrex Gly | 19,50 | 20,00 |
| Ethylhexylglycerin (Octoxyglycerin) | 0,30 | 0,20 |
| 4-Methyl-4-phenyl-2-pentanol (Vetikol) | 0,25 | 0,10 |
| Parfümöl A2 aus Beispiel 4 | 1,00 | - |
| Parfümöl C2 aus Beispiel 6 | - | 0,80 |

### Beispiel 15: Aerosolspray

| **Komponente** | **Gew.-%** | **Gew.-%** | **Gew.-%** |
|---|---|---|---|
| Octyldodecanol | 0,50 | - | 0,50 |
| Phenoxyethanol | - | - | 0,30 |
| 1,2-Pentandiol | 1,00 | 1,00 | 0,50 |
| 1,2-Hexandiol | 0,25 | 0,15 | 0,25 |
| 1,2-Octandial | 0,25 | 0,25 | 0,25 |
| Farnesol | - | 0,25 | 0,15 |
| Ethylhexylglycerin (Octoxyglycerin) | 0,50 | 0,30 | 0,50 |
| Parfümöl A2 aus Beispiel 4 | 0,80 | - | 0,50 |
| Parfümöl H2 aus Beispiel 12 | - | 1,15 | 0,50 |
| Ethanol | Ad 100 | Ad 100 | Ad 100 |

Die nach Zusammenmischen der jeweils angegebenen Komponenten erhaltene Mischung wird mit einem Propan-Butan-Gemisch (2:7) im Gewichtsverhältnis 2 : 3 in einen Aerosolbehälter abgefüllt.

### Beispiel 16: Haarconditioner mit UV-Schutz

| **Komponente** | **INCI Name** | **Gew.-%** | **Gew.-%** |
|---|---|---|---|
| Lanette O | Cetearyl Alcohol | 4,00 | 4,00 |
| Dragoxat 89 | Ethylhexyl Isononanoate | 4,00 | 4,00 |
| Emulsiphos | Potassium Cetyl Phosphate, Hydrogenated Palm Glycerides | 0,50 | 0,50 |
| Natrosol 250 HR | Hydroxyethylcellulose | 0,25 | 0,25 |
| Neo Heliopan Hydro | Phenylbenzimidazole Sulfonic Acid | 2,00 | 2,00 |
| L- Arginin | Arginine | 1,20 | 1,20 |
| Benzophenon-4 | Benzophenone-4 | 0,50 | 0,50 |
| Neo Heliopan AP | Disodium Phenyl Dibenzimidazole | 0,50 | 1,00 |
| | Tetrasulfonate | | |
| Edeta BD | Disodium EDTA | 0,05 | 0,05 |
| Dragocide Liquid | Phenoxyethanol (and) Methylparaben (and) Butyparaben (and) Ethyparaben (and) Propylparaben | 0.80 | 0,80 |
| Dow Corning 949 Cationic Emulsion | Amodimethicone, Cetrimonium Chloride, Trideceth-12 | 2,00 | 2,00 |
| Dow Corning 5200 | Laurylmethicone Copolyol | 0,50 | 0,50 |
| Parfümöl B2 aus Beispiel 5 | Parfum | 0,95 | - |
| Parfümöl H2 aus Beispiel 12 | Parfum | | 1,25 |
| Wasser | Water (Aqua) | Ad 100 | Ad 100 |

### Beispiel 17: Sonnenschutzspray

| **Teil** | **Rohstoffe** | **INCI Bezeichnung** | **Gew.-%** |
|---|---|---|---|
| **A** | Wasser, demineralisiert | Water (Aqua) | 69,50 |
| | Glycerin | Glycerin | 4,00 |
| | 1,3 Butylenglykol | Butylene Glycol | 5,00 |
| | D-Panthenol | Panthenol | 0,50 |
| | Lara Care A-200 | Galactoarabinan | 0,25 |
| B | Baysiloneöl M 10 | Dimethicone | 1,00 |
| | Edeta BD | Disodium EDTA | 0,10 |
| | Copherol 1250 | Tocopheryl Acetate | 0,50 |
| | Cetiol OE | Dicaprylyl Ether | 3,00 |
| | Neo Heliopan^{®} HMS | Homosalate | 5,00 |
| | Neo Heliopan^{®} AV | Ethylhexyl Methoxycinnamate | 6,00 |
| | Neo Heliopan^{®} 357 | Butyl Methoxydibenzoylmethane | 1,00 |
| | Corapan TQ | Diethylhexylnaphthalate | 2,00 |
| | Alpha Bisabolol | Bisabolol | 0,10 |
| | Pemulen TR-2 | Acrylates/C10-30 Alkyl Acrylate Crosspolymer | 0,25 |
| C | Phenoxyethanol | Phenoxyethanol | 0,70 |
| | Solbrol M | Methylparaben | 0,20 |
| | Solbrol P | Propylparaben | 0,10 |
| D | NaOH, 10 %ig | Sodium Hydroxide | 0,60 |
| E | Parfümöl D2 aus Beispiel 7 | Fragrance (Parfum) | 0,20 |

Herstellungsverfahren: Lara Care A-200 unter Rühren in den anderen Bestandteilen von Teil A lösen. Alle Rohstoffe des Teils B (ohne Pemulen) einwiegen und die kristallinen Substanzen unter Erwärmen lösen. Pemulen eindispergieren. Teil B zu Teil A geben und 1 Minute homogenisieren. Teile C-E zugeben und nochmals 1-2 Minuten mit dem Ultra Turrax homogenisieren.

### Beispiel 18: Sonnenschutz Softcreme (W/O), Lichtschutzfaktor (SPF) 40

| **Teil** | **Rohstoffe** | **INCI Bezeichnung** | **Gew.-%** |
|---|---|---|---|
| **A** | Dehymuls PGPH | Polyglyceryl-2 Dipolyhydroxystearate | 5,00 |
| | Copherol 1250 | Tocopheryl acetate | 0,50 |
| | Permulgin 3220 | Ozokerite | 0,50 |
| | Zinkstearat | Zinc Stearate | 0,50 |
| | Tegosoft TN | C12-15 Alkyl Benzoate | 10,00 |
| | Neo Heliopan^{®} E1000 | Isoamyl-p-Methoxycinnamate | 2,00 |
| | Neo Heliopan^{®} 303 | Octocrylene | 5,00 |
| | Neo Heliopan^{®} MBC | 4-Methylbenzylidene Camphor | 3,00 |
| | Zinkoxid neutral | Zinc Oxide | 5,00 |
| **B** | Wasser, destilliert | Water (Aqua) | Ad 100 |
| | EDETA BD | Disodium EDTA | 0,10 |
| | Glycerin | Glycerin | 4,00 |
| | Phenoxyethanol | Phenoxyethanol | 0,70 |
| | Solbrol M | Methylparaben | 0,20 |
| | Solbrol P | Propylparaben | 0,10 |
| | Magnesiumsulfat | Magnesium Sulfate | 0,50 |
| **C** | Parfümöl B2 aus Beispiel 5 | Parfum (Fragrance) | 0,30 |

Herstellungsverfahren: Teil A auf ca. 85 °C erhitzen. Teil B (ohne Zinkoxid) auf ca. 85 °C erhitzen; Zinkoxid mit dem Ultra Turrax eindispergieren, B zu A geben. Unter Rühren abkühlen lassen. Teil C zugeben und anschließend homogenisieren.

### Beispiel 19: Sonnenschutzmilch (W/O)

| **Teil** | **Rohstoffe** | **INCI Bezeichnung** | **Gew.-%** |
|---|---|---|---|
| **A** | Dehymuls PGPH | Polyglyceryl-2 Dipolyhydroxystearate | 3,00 |
| | Bienenwachs 8100 | Beeswax | 1,00 |
| | Monomuls 90-0-18 | Glyceryl Oleate | 1,00 |
| | Zinkstearate | Zinc stearate | 1,00 |
| | Cetiol SN | Cetearyl Isononanoate | 5,00 |
| | Cetiol OE | Dicaprylyl Ether | 5,00 |
| | Tegosoft TN | C12-15 Alkyl Benzoate | 4,00 |
| | Vitamin E | Tocopherol | 0,50 |
| | Solbrol P | Propylparaben | 0,10 |
| | Neo Heliopan^{®} OS | Ethylhexyl Salicylate | 5,00 |
| | Neo Heilopan^{®} AV | Ethylhexyl Methoxycinnamate | 7,50 |
| | Uvinul^{®} T150 | Ethylhexyl Triazone | 1,50 |
| **B** | Wasser, destilliert | Water (Aqua) | Ad 100 |
| | Trilon BD | Disodium EDTA | 0,10 |
| | Glycerin | Glycerin | 5,00 |
| | Solbrol M | Methylparaben | 0,20 |
| | Phenoxyethanol | Phenoxyethanol | 0,70 |
| | Neo Heliopan^{®} AP 10%ige Lösung, neutralisiert mit NaOH | Disodium Phenyl Dibenzimidazole Tetrasulfonate | 15,00 |
| **C** | Parfümöl C2 aus Beispiel 6 | Parfum (Fragrance) | 0,25 |
| | Alpha Bisabolol | Bisabolol | 0,10 |

Herstellungsverfahren: Teil A auf ca. 85 °C erhitzen. Teil B auf ca. 85 °C erhitzen. B zu A geben, unter Rühren abkühlen lassen. Teil C zugeben und anschließend homogenisieren.

### Beispiel 20: Permanent-Haarfärbemittel

| **Rohstoffe A - Haarfärbungsbase** | **Gew.-%** |
|---|---|
| Natrium Myreth Sulfat (z. B. Texapon K14 S/K, Cognis) | 2,80 |
| Linoleamidopropyl PG-dimonium chlorid phosphat (Arlasilk Phospholipid EFA, Uniqema) | 1,00 |
| Caprylyl- / Capryl glucosid (Plantacare 810 UP, Cognis) | 2,00 |
| Natrium Laureth-6 Carboxylat (Akypo Soft 45 NV, Kao) | 10,00 |
| Cetearylalkohol | 8,00 |
| Octyldodecanol | 1,00 |
| Ceteareth-12 | 0,50 |
| Ceteareth-20 | 0,50 |
| KOH, 50%ig in Wasser | 0,70 |
| Toluol-2,5-diamine sulfat (oxidierende Farbstoff) | 0,90 |
| Resorcin | 0,20 |
| m-aminophenol | 0,06 |
| 4-chlororesorcin | 0,15 |
| Ascorbinsäure | 0,10 |
| Natriumsulfit | 0,15 |
| Ammoniak, 25% in Wasser | 6,00 |
| Etidronsäure | 0,20 |
| Polyquaternium-2 (Mirapol A15, Rhodia) | 0,20 |
| Parfümöl G2 aus Beispiel 10 | 0,30 |
| Wasser | 65,24 |

| **Rohstoffe B - Entwickler** | **Gew.-%** |
|---|---|
| Cetostearylalkohol | 8.00 |
| Ceteareth-20 | 2.50 |
| Steartrimonium Chlorid (Dehyquart B, Cognis) | 1.00 |
| 2,6-dicarboxypyridin | 0.10 |
| Paraffinöl | 0.30 |
| Etidronsäure | 0.40 |
| Propylenglykol | 0.40 |
| Natriumbenzoat | 0.04 |
| Wasserstoffperoxid, 50% in Wasser | 12.00 |
| KOH, 50% in Water, zugegeben bis pH = 3.5 | q.s. |
| Wasser | To 100 |

Anwendung: Die erfindungsgemäße Haarfärbungsbasis (Teil A) und der erfindungsgemäße Entwickler (Teil B) werden in einem Gewichtsverhältnis von 1:1 Verhältnis zusammen gerührt und auf die Haare aufgetragen.

### Beispiel 21: Haarfärbungscreme für eine blonde Farbtönung

| **Teil** | **Material** | **Hersteller** | **INCI-Name** | **Gew.-%** |
|---|---|---|---|---|
| **A** | Oxowax | LCW, Frankreich | Cethyl alcohol, Oleyl alcohol, Cetearyl alcohol, Stearic acid | 21,00 |
| | Volpo CS 25 | Croda GmbH, Deutschland | Ceteareth25 | 4,00 |
| | Berol175 | Brenntag Eurochem GmbH, Deutschland | Laureth-8 | 10,00 |
| | Lanette® E | Cognis Dtl. GmbH, Deutschland | Sodium Cetearyl Sulfate | 1,00 |
| | Covaquat 16 | LCW, Frankreich | Polyquaternium-6 | 4,00 |
| | Wasser | | Water (Aqua) | 49,30 |
| **B** | Ammoniak (20% in Wasser) | Merk Eurolab | Ammonia | 10,20 |
| **C** | Parfümöl D2 aus Beispiel 7 | Symrise | Parfum | 0,50 |

Herstellungsverfahren: Teil A, B und C außer Covaquat 16 zusammen mischen und 20 Minuten bei 80 °C erhitzen. Nachdem das Produkt homogen ist, abkühlen lassen. Covaquat 16 bei 50 °C dazugeben. Wenn das Produkt anfängt zu verdicken die Rührung stoppen. Bei Raumtemperatur NH₄OH dazugegeben und die Mischung bis zur Homogenisierung rühren.

### Beispiel 22: Lufterfrischer Aerosolspray, aus Wasserbasis (w/o Alkohol)

| **Material** | **Hersteller** | **INCI-Name** | **Gew.-%** |
|---|---|---|---|
| Imbentin C/125/030 | Symrise | C12-15 pareth-3 | 0,25 |
| Imbentin AG/100/080 | Symrise | deceth-8, Ziegler alcohol | 1,00 |
| Parfümöl A2 aus Beispiel 4 | Symrise | Parfum | 1,25 |
| Wasser, demineralisiert | | Aqua | 22,50 |
| Treibgas 4,2 bar | | | 75,00 |

Herstellungsverfahren: Alle Rohstoffe in der angegebenen Reihenfolge zusammenmischen.

### Beispiel 23: flüssige Lufterfrischer Pumpenspray

| **Material** | **Hersteller** | **INCI-Name** | **Gew.-%** |
|---|---|---|---|
| Wasser, demineralisiert | | Aqua | 90,70 |
| Ethanol 96% | Symrise | Ethanol | 4,00 |
| Empilan KCL 11/90 | Stockmeier Chemie GmbH&Co. KG | Alcohol C12-1511EO | 3,00 |
| Isopropanol | Symrise | Propan-2-ol | 1,00 |
| Natriumhydrogencarbonat p.A., | Symrise | Sodiumbicarbonat | 0,20 |
| Parmetol A 26 | Hoesch Julius | Mixture of 5-Chloro-2-methyl-2*H*-isothiazol-3-one und 2-Methyl-2*H*-isothiazol-3-one | 0,10 |
| Parfümöl F2 aus Beispiel 9 | Symrise | Parfum | 1,00 |

Herstellungsverfahren: Alle Rohstoffe in der angegebenen Reihenfolge zusammen mischen. pH-Wert anpassen.

### Beispiel 24: deodorierende Lufterfrischer Pumpe/Docht 5% fragrance

| **Teil** | **Material** | **Hersteller** | **INCI-Name** | **Gew.-%** |
|---|---|---|---|---|
| **A** | Wasser, demineralisiert | | Aqua | 67,32 |
| | Sequion 40 NA 32 | Polygon Chemie | | 1,25 |
| | Triethanolamin pur C | Symrise | Triethanolamine | 0,30 |
| | Rewoderm S 1333 | Goldschmidt AG | DiNa-Ricinoleamido MEA-Sulfosuccinat | 2,33 |
| | Tego Sorb Conc. 50 | Evonik | Alcohol C12-14, ethoxylated (2-5 EO) + Zn-Ricinoleat | 0,50 |
| | Ethanol 96% | Symrise | Ethanol | 20,00 |
| **B** | Parfümöl E2 aus Beispiel 8 | Symrise | Parfum | 5,00 |
| | Lösungsvermittler | Symrise | | 3,00 |
| **C** | Milchsäure | Symrise | Propanoic acid, 2-hydroxypropionic acid, Lactol | 0,30 |

Herstellungsverfahren: Materialien des Teils A in der angegebenen Reihenfolge zusammen mischen. Materialien des Teil B zusammen mischen. Wenn Teil A klar ist, Teil B dazugeben. Anschließend Teil C zugeben und rühren bis die Mischung homogen ist.

### Beispiel 25: Kerze

| **Material** | **Gew.-%** |
|---|---|
| Kerzenwachs | 95,00 |
| Parfümöl D2 aus Beispiel 7 | 5,00 |

Herstellungsverfahren: Das Kerzenwachs schmelzen lassen und rühren. Das Parfümöl dazugeben, gut rühren. In die gewünschte Form gießen.

### Beispiel 26: WC-Stein

| **Teil** | **Material** | **Hersteller** | **INCI-Name** | **Gew.-%** |
|---|---|---|---|---|
| **A** | Imbentin C/125/200 | Dr. W. Kolb AG | C12/15 pareth-20 | 4,00 |
| | Rewomid C 212 | Goldschmidt AG | Cocamide MEA | 6,00 |
| | Marlon ARL | Sasol GmbH | C10-13, ABS-Na, Pulver | 42,00 |
| **B** | Natriumsulfat pharm. (E514) | Symrise | Sodium sulfate | 42,00 |
| | Parfümöl F2 aus Beispiel 9 | Symrise | Parfum | 6,00 |

Herstellungsverfahren: Teil A schmelzen lassen. Teil B dazumischen mit Hilfe eines Mischkneters. Im Extruder bei 35-40 °C zu einem WC-Stein formen.

### Beispiel 27: Intensität

Der Intensitätstest ist ein Test zur Bestimmung der sensorischen Stärke eines Stoffes auf Riechstreifen.

2-(3-Methylbutoxy)acetaldehyd (Formel (la)) wird in Form einer 50 Gew.-%igen Lösung in einem Lösungsmitel ausgewählt aus der Gruppe bestehend aus Triethylcitrat (TEC), Diethylphthalat (DEP), Benylbenzoat, Isopropylmyristat (IPM), Dioctyladipat (DOA) und Dipropylenglycol (DPG) jeweils auf einen codierten Riechstreifen getaucht, und von einem Probandenpanel (5 oder mehr Personen) wird die Intensität der Maiglöckchen-Geruchsnote auf einer Skala von 1 (geruchlos) bis 9 (sehr stark) beurteilt. Aus den einzelnen Messwerten werden Mittelwerte berechnet, die auf ganze Zahlen gerundet werden. Zum Vergleich werden auch die reinen Lösungsmittel Triethylcitrat (TEC), Diethylphthalat (DEP), Benylbenzoat, Isopropylmyristat (IPM), Dioctyladipat (DOA) bzw. Dipropylenglycol (DPG) jeweils auf einen Riechstreifen getaucht und hinsichtlich der Intensität der Maiglöckchen-Note bewertet.

Dabei wurde festgestellt, dass die Intensität der Maiglöckchen-Note bei gleicher Konzentration von 2-(3-Methylbutoxy)acetaldehyd (Formel (Ia)) deutlich stärker und in einigen Fällen sogar doppelt so stark ist, wenn das Lösungsmittel Dipropylenglykol (DPG) durch ein Lösungsmittel aus der Gruppe bestehend aus Triethylcitat (TEC), Diethylphthalat (DEP), Benylbenzoat, Isopropylmyristat (IPM) und Dioctyladipat (DOA) ersetzt wird.

| Probe | Intensität der Maiglöckchen-Note |
|---|---|
| Triethylcitrat (TEC) | 1 |
| Dipropylenglykol (DPG) | 1 |
| Diethylphthalat (DEP) | 1 |
| Benzylbenzoat | 1 |
| Isopropylmyristat (IPM) | 1 |
| Dioctyladipat (DOA) | 1 |
| 2-(3-Methylbutoxy)acetaldehyd (Formel (Ia)) 50%ig in DPG | 3 |
| 2-(3-Methylbutoxy)acetaldehyd (Formel (Ia)) 50%ig in TEC | 6 |
| 2-(3-Methylbutoxy)acetaldehyd (Formel (Ia)) 50%ig in DEP | 5 |
| 2-(3-Methylbutoxy)acetaldehyd (Formel (Ia)) 50%ig in Benzylbenzoat | 5 |
| 2-(3-Methylbutoxy)acetaldehyd (Formel (Ia)) 50%ig in (IPM) | 5 |
| 2-(3-Methylbutoxy)acetaldehyd (Formel (Ia)) 50%ig in (DOA) | 6 |

## Patentansprüche

1. Verwendung einer Verbindung der Formel (I) oder einer Mischung enthaltend eine oder mehrere Verbindungen der Formel (I) oder einer Mischung bestehend aus zwei oder mehreren Verbindungen der Formel (I), 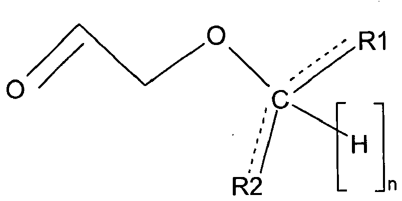 worin gilt:
n ist 0 oder 1, wobei
(i) wenn n = 1
die beiden Paare aus einer durchgezogenen und einer gestrichelten Linie in der Formel (I) jeweils Einfachbindungen darstellen
und
a) R1 und R2 jeweils eine unverzweigte gesättigte Alkylgruppe darstellen, wobei die Gesamtanzahl der Kohlenstoffatome in den Gruppen R1 und R2 4 bis 9 beträgt
oder
b) R1 und R2 gemeinsam mit dem zwischen ihnen angeordneten Kohlenstoffatom einen Cyclohexylring 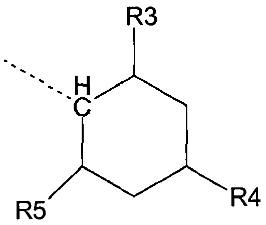 bilden,
wobei die gestrichelte Linie die Bindung darstellt, die den Cyclohexylring mit dem in Formel (I) benachbarten Sauerstoffatom verbindet, und
wobei R3, R4 und R5 unabhängig voneinander ausgewählt sind aus der Gruppe bestehend aus Wasserstoff, Methyl, Ethyl, Isopropyl und tert-Butyl und wobei die Gesamtanzahl der Kohlenstoffatome des Cyclohexylrings und der Gruppen R3, R4 und R5 6 bis 10 beträgt
oder
c) R1 Wasserstoff ist und R2
c1) eine verzweigte gesättigte Alkylgruppe mit 4 bis 9 Kohlenstoffatomen ist
oder
c2) ein Cyclohexylring 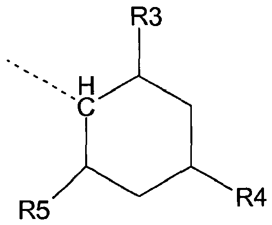 ist, wobei die gestrichelte Linie die Bindung darstellt, die den Cyclohexylring mit dem in Formel (I) benachbarten Kohlenstoffatom verbindet, und
wobei R3, R4 und R5 unabhängig voneinander ausgewählt sind aus der Gruppe bestehend aus Wasserstoff, Methyl, Ethyl und Isopropyl und die Gesamtanzahl der Kohlenstoffatome des Cyclohexylrings und der Gruppen R3, R4 und R5 6 bis 9 beträgt
oder
c3) ein Phenylring 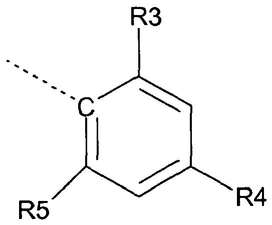 ist, wobei die gestrichelte Linie die Bindung darstellt, die den Phenylring mit dem in Formel (I) benachbarten Kohlenstoffatom verbindet, und
wobei R3, R4 und R5 unabhängig voneinander ausgewählt sind aus der Gruppe bestehend aus Wasserstoff, Methyl, Ethyl und Isopropyl und die Gesamtanzahl der Kohlenstoffatome des Phenylrings und der Gruppen R3, R4 und R5 6 bis 9 beträgt
und
(ii) wenn n = 0
die beiden Paare aus einer durchgezogenen und einer gestrichelten Linie in der Formel (I) jeweils aromatische Bindungen darstellen
und
(d) R1 und R2 gemeinsam mit dem zwischen ihnen angeordneten Kohlenstoffatom einen Phenylring 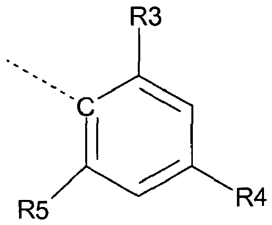 bilden,
wobei die gestrichelte Linie die Bindung darstellt, die den Phenylring mit dem in Formel (I) benachbarten Sauerstoffatom verbindet, und
wobei R3, R4 und R5 unabhängig voneinander ausgewählt sind aus der Gruppe bestehend aus Wasserstoff, Methyl, Ethyl, Isopropyl und tert-Butyl und die Gesamtanzahl der Kohlenstoffatome des Phenylrings und der Gruppen R3, R4 und R5 6 bis 10 beträgt,
oder einer Verbindung ausgewählt aus der Gruppe bestehend aus
3.) 2-(1,1-Dimethylpropoxy)acetaldehyd 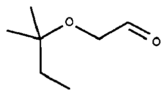
4.) 2-(1,3-Dimethylbutoxy)acetaldehyd 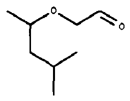
6.) 2-[(1,5-Dimethylhexyl)oxy]acetaldehyd 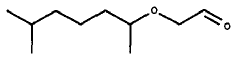
7.) 2-(1,1-Dimethylhexoxy)acetaldehyd 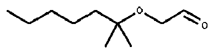
8.) 2-[(2-Ethylhexyl)oxy]acetaldehyd 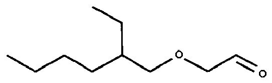
- als Riechstoff mit einer Maiglöckchen-Note
und/oder
- als Mittel zum Erhöhen der Substantivität und/oder der Retention einer Riechstoffzubereitung
und/oder
- als Fixateur.

2. Verwendung nach Anspruch 1, wobei
eine oder mehrere Verbindungen der Formel (I) wie in Anspruch 1 definiert verwendet werden zum Vermitteln, Modifizieren oder Verstärken einer Maiglöckchen-Geruchsnote und
(i) einer, mehrerer oder sämtlicher Geruchsnoten ausgewählt aus der Gruppe bestehend aus blumig, fruchtig, wässrig, grün und aldehydig und/oder
(ii) eines, mehrerer oder sämtlicher Eindrücke ausgewählt aus der Gruppe bestehend aus natürlich, frisch, pflegend, sanft, warm, komplex und strahlend.

3. Verwendung nach Anspruch 1 oder 2, **dadurch gekennzeichnet, dass** die verwendete Verbindung der Formel (I) oder eine, mehrere oder sämtliche der Verbindungen der Formel (I) in der verwendeten Mischung ausgewählt sind aus der Gruppe bestehend aus Verbindungen, in denen
- n = 1 ist, R1 Wasserstoff ist und R2 eine verzweigte gesättigte Alkylgruppe 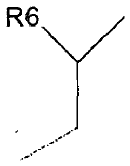 mit insgesamt 4 bis 9 Kohlenstoffatomen ist, in der R6 eine verzweigte oder unverzweigte gesättigte Alkylgruppe mit 1 bis 6 Kohlenstoffatomen ist und die gestrichelte Linie die Bindung darstellt, die die verzweigte gesättigte Alkylgruppe R2 mit dem in Formel (I) benachbarten Kohlenstoffatom verbindet, oder
- n = 1 ist und R1 und R2 gemeinsam mit dem zwischen ihnen angeordneten Kohlenstoffatom einen Cyclohexylring bilden wie oben definiert, R3 und R5 Wasserstoff sind und R4 ausgewählt ist aus der Gruppe bestehend aus Methyl, Ethyl und Isopropyl und tert-Butyl, oder
- n = 0 ist und R1 und R2 gemeinsam mit dem zwischen ihnen angeordneten Kohlenstoffatom einen Phenylring bilden wie oben definiert, R3 und R5 Wasserstoff sind und R4 ausgewählt ist aus der Gruppe bestehend aus Methyl, Ethyl und Isopropyl und tert-Butyl, oder
- n = 1 ist, R1 Methyl ist und R2 eine unverzweigte gesättigte Alkylgruppe mit 4 bis 6 Kohlenstoffatomen ist.

4. Verwendung nach Anspruch 1 oder 2, **dadurch gekennzeichnet, dass** die verwendete Verbindung der Formel (I) oder eine, mehrere oder sämtliche der Verbindungen der Formel (I) in der verwendeten Mischung ausgewählt sind aus der Gruppe bestehend aus Verbindungen, in denen
- n = 1 ist, R1 Wasserstoff ist und R2 eine verzweigte gesättigte Alkylgruppe 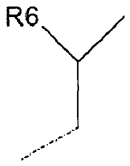 mit insgesamt 4 bis 9 Kohlenstoffatomen ist, in der R6 eine verzweigte oder unverzweigte gesättigte Alkylgruppe mit 1 bis 6 Kohlenstoffatomen ist und die gestrichelte Linie die Bindung darstellt, die die verzweigte gesättigte Alkylgruppe R2 mit dem in Formel (I) benachbarten Kohlenstoffatom verbindet, oder
- n = 1 ist, R1 Methyl ist und R2 eine unverzweigte gesättigte Alkylgruppe mit 4 bis 6 Kohlenstoffatomen ist.

5. Verwendung nach einem der Ansprüche 1 bis 3, **dadurch gekennzeichnet, dass** die verwendete Verbindung der Formel (I) oder eine, mehrere oder sämtliche der Verbindungen der Formel (I) in der verwendeten Mischung ausgewählt sind aus der Gruppe bestehend aus
- 2-(3-Methylbutoxy)acetaldehyd (Formel (la)) (2-Isopentyloxyacetaldehyd) 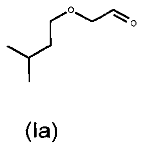
- 2-(4-Isopropylcyclohexyloxy)acetaldehyd (Formel (Ib)) 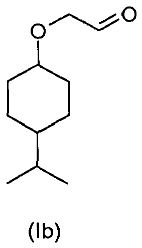
- 2-(4-Isopropylphenoxy)acetaldehyd (Formel (Ic)) 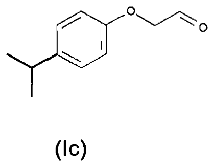
- 2-(3,5,5-Trimethylhexoxy)acetaldehyd (Formel (Id)) 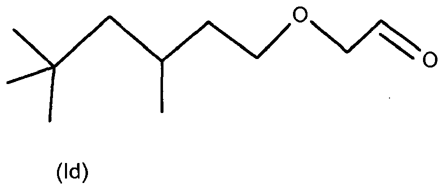
- 2-[(1-Methylheptyl)oxy]acetaldehyd (Formel (Ie))

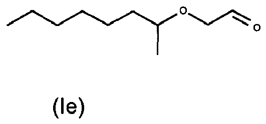

6. Verbindung ausgewählt aus der Gruppe bestehend aus:
2-((4-Isopropylcyclohexyloxy)acetaldehyd (Formel (Ib)) 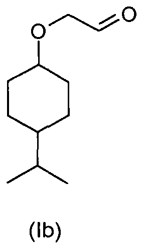 und
2-((3,5,5-trimethylhexoxy)acetaldehyd (Formel (Id))

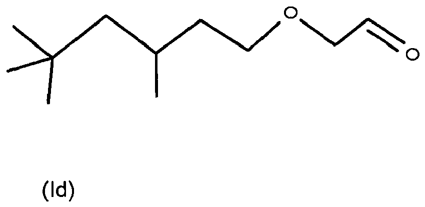

7. 1-(2,2-Diethoxyethoxy)-4-isopropylcyclohexan (Formel (IIb)

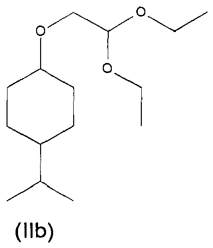

8. Mischung enthaltend eine oder mehrere Verbindungen der Formel (I) wie in einem der vorhergehenden Ansprüche definiert, vorzugsweise eine oder mehrere Verbindungen der Formel (I) wie in einem der Ansprüche 3 bis 5 definiert wobei die Mischung
- einen, 2, 3, 4, 5, 6, 7, 8, 9, 10 oder mehr weitere Riechstoffe enthält, die keine Verbindungen der Formel (I) sind,
und/oder
- ein oder mehrere Lösungsmittel enthält, die ausgewählt sind aus der Gruppe bestehend aus den Trialkylcitraten (vorzugsweise Triethylcitrat, Trimethylcitrat, Tripropylcitrat, Triisopropylcitrat, Tributylcitrat, Triisobutylcitrat oder Tri-sec-butylcitrat), den Dialkylphthalaten (vorzugsweise Dimethylphthalat, Diethylphthalat oder Dibutylphthalat), Benzylbenzoat, Isopropylmyristat und Dioctyladipat,
und/oder
- ein Produkt ist ausgewählt ist aus der Gruppe bestehend aus Wasch- und Reinigungsmitteln, Hygiene- oder Pflegeprodukten, insbesondere aus dem Bereich der Körper- und Haarpflege, der Kosmetik und des Haushalts, Fine Fragrances (Parfüme), Parfümölen, Air Care-Produkten (insbesondere Kerzen), Lufterfrischern, Mitteln zum Reinigen, Behandeln und/oder Pflegen von Oberflächen und textilen Fasern.

9. Mischung nach Anspruch 8, **dadurch gekennzeichnet, dass** die Mischung einen, 2, 3, 4, 5, 6, 7, 8, 9, 10 oder mehr weitere Riechstoffe enthält, die keine Verbindungen der Formel (I) sind, und die Gesamtmenge der Verbindungen der Formel (I) wie in einem der vorhergehenden Ansprüche definiert im Bereich von 0,0001 bis 40 Gew.-% liegt, vorzugsweise im Bereich von 0,001 bis 25 Gew.-%, jeweils bezogen auf das Gesamtgewicht der Mischung.

10. Mischung nach Anspruch 8, **dadurch gekennzeichnet, dass** die Mischung ein oder mehrere Lösungsmittel enthält, die ausgewählt sind aus der Gruppe der Trialkylcitrate, und die Gesamtmenge der Verbindungen der Formel (I) wie in einem der vorhergehenden Ansprüche definiert im Bereich von 0,0001 bis 40 Gew.- % liegt, vorzugsweise im Bereich von 0,001 bis 25 Gew.-%, jeweils bezogen auf das Gesamtgewicht der Mischung.

11. Mischung nach Anspruch 8 oder 10, **dadurch gekennzeichnet, dass** das oder eines der Lösungsmittel ausgewählt ist aus der Gruppe bestehend aus Triethylcitrat, Diethylphthalat, Benzylbenzoat, Isopropylmyristat und Dioctyladipat.

12. Mischung nach Anspruch 8, **dadurch gekennzeichnet, dass** die Mischung ein Produkt ist ausgewählt aus der Gruppe bestehend aus Wasch- und Reinigungsmitteln, Hygiene- oder Pflegeprodukten, insbesondere aus dem Bereich der Körper- und Haarpflege, der Kosmetik und des Haushalts, Fine Fragrances (Parfüme), Air Care-Produkten (insbesondere Kerzen), Lufterfrischern, Mitteln zum Reinigen, Behandeln und/oder Pflegen von Oberflächen und textilen Fasern und die Gesamtmenge der Verbindungen der Formel (I) wie in einem der vorhergehenden Ansprüche definiert im Bereich von 0,0001 bis 5 Gew.-% liegt, vorzugsweise im Bereich von 0,001 bis 2,5 Gew.-%, jeweils bezogen auf das Gesamtgewicht des Produkts.

13. Verfahren zum Vermitteln und/oder Verstärken
(i) einer Maiglöckchen-Geruchsnote
sowie (ii) gegebenenfalls
(ii)a) einer, mehrerer oder sämtlicher Geruchsnoten ausgewählt aus der Gruppe bestehend aus blumig, fruchtig, wässrig, grün und aldehydig und/oder
(ii)b) eines, mehrerer oder sämtlicher Eindrücke ausgewählt aus der Gruppe bestehend aus natürlich, frisch, pflegend, sanft, warm, komplex und strahlend;
umfassend den folgenden Schritt:
Mischen oder in Kontakt bringen
- einer oder mehrerer Verbindungen der Formel (I) wie in einem der vorhergehenden Ansprüche definiert, vorzugsweise eine oder mehrere Verbindungen der Formel (I) wie in einem der Ansprüche 3 bis 5 definiert, oder
- einer Mischung nach einem der Ansprüche 8 bis 12
in einer sensorisch wirksamen Menge mit einem Erzeugnis.

14. Verfahren nach Anspruch 13 zum Versehen von Haut, Haaren, Oberflächen oder textilen Fasern mit
(i) einer Maiglöckchen-Geruchsnote
sowie (ii) gegebenenfalls
(ii)a) einer, mehrerer oder sämtlicher Geruchsnoten ausgewählt aus der Gruppe bestehend aus blumig, fruchtig, wässrig, grün und aldehydig
und/oder
(ii)b) eines, mehrerer oder sämtlicher Eindrücke ausgewählt aus der Gruppe bestehend aus natürlich, frisch, pflegend, sanft, warm, komplex und strahlend;
umfassend folgende Schritte:
- Bereitstellen
- einer oder mehrerer Verbindungen der Formel (I) wie in einem der vorhergehenden Ansprüche definiert, vorzugsweise eine oder mehrere Verbindungen der Formel (I) wie in einem der Ansprüche 3 bis 5 definiert, oder
- einer Mischung nach einem der Ansprüche 8 bis 12,
- Applizieren der Verbindung(en) der Formel (I) bzw. der Mischung oder des parfümierten Produkts auf Haut, Haare, Oberflächen oder textile Fasern.

## Claims

1. Use of a compound of formula (I) or a mixture comprising one or more compounds of formula (I) or of a mixture consisting of two or more compounds of formula (I), 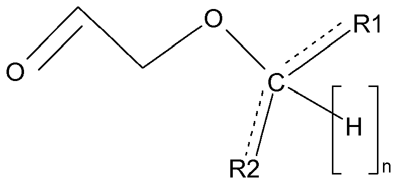 wherein the following applies:
n is 0 or 1, wherein
(i) if n = 1
the two pairs of a drawn-through and a dotted line in formula (I) in each case denote single bonds
and
a) R1 and R2 in each case denote an unbranched saturated alkyl group, wherein the total number of carbon atoms in the groups R1 and R2 is 4 to 9
or
b) R1 and R2 together with the carbon atom located in between form a cyclohexyl ring, 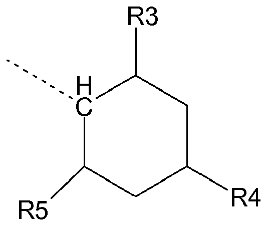 wherein the dotted line represents the bond, which joins the cyclohexyl ring to the adjacent oxygen atom of formula (I), and
wherein R3, R4 and R5 independent of each other are selected from the group consisting of hydrogen, methyl, ethyl, isopropyl and tert-butyl and wherein the total number of carbon atoms of the cyclohexyl ring and the groups R3, R4 and R5 is 6 to 10
or
c) R1 is hydrogen and R2 is
c1) a branched saturated alkyl group with 4 to 9 carbon atoms
or
c2) a cyclohexyl ring, 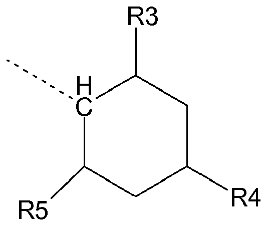 wherein the dotted line represents the bond, which joins the cyclohexyl ring to the adjacent carbon atom of formula (I), and
wherein R3, R4 and R5 independent of each other are selected from the group consisting of hydrogen, methyl, ethyl and isopropyl and the total number of carbon atoms of the cyclohexyl ring and the groups R3, R4 and R5 is 6 to 9
or
c3) a phenyl ring, 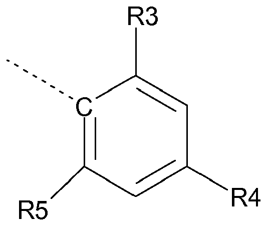 wherein the dotted line represents the bond, which joins the phenyl ring to the adjacent carbon atom of formula (I), and
wherein R3, R4 and R5 independent of each other are selected from the group consisting of hydrogen, methyl, ethyl and isopropyl and the total number of carbon atoms of the phenyl ring and the groups R3, R4 and R5 is 6 to 9
and
(ii) if n = 0
the two pairs of a drawn-through and a dotted line in formula (I) in each case denote single bonds
and
(d) R1 and R2 together with the carbon atom located in between form a phenyl ring, 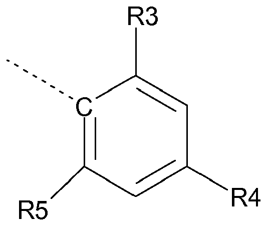 wherein the dotted line represents the bond, which joins the phenyl ring to the adjacent oxygen atom of formula (I),
wherein R3, R4 and R5 independent of each other are selected from the group consisting of hydrogen, methyl, ethyl, isopropyl and tert-butyl and the total number of carbon atoms of the phenyl ring and the groups R3, R4 and R5 is 6 to 10,
or of a compound selected from the group consisting of
3.) 2-(1,1-dimethylpropoxy)acetaldehyde 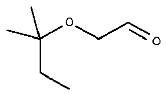
4.) 2-(1,3-dimethylbutoxy)acetaldehyde 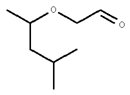
6.) 2-[(1,5-dimethylhexyl)oxy]acetaldehyde 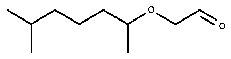
7.) 2-(1,1-dimethylhexoxy)acetaldehyde 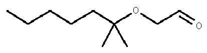
8.) 2-[(2-ethylhexyl)oxy]acetaldehyde 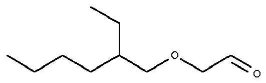
- as fragrance with a lily of the valley note
and/or
- as means to increase the substantivity and/or the retention of a fragrance preparation
and/or
- as fixative.

2. Use according to claim 1, wherein
one or more compounds of formula (I) as defined in claim 1 are used to convey, modify or enhance a lily of the valley fragrance note and
(i) one, more or all fragrance notes selected from the group consisting of flowery, fruity, watery, green and aldehyde
and/or
(ii) one, more or all impressions selected from the group consisting of natural, fresh, nurturing, gentle, warm, complex and radiant.

3. Use according to claim 1 or 2, **characterized in that** the compound of formula (I) used or one, more or all compounds of formula (I) in the mixture used are selected from the group consisting of compounds, in which
- n = 1, R1 is hydrogen and R2 is a branched saturated alkyl group 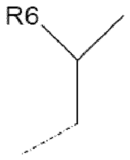 with 4 to 9 carbon atoms in total, in which R6 is a branched or unbranched saturated alkyl group with 1 to 6 carbon atoms and the dotted line represents the bond, which joins the branched saturated alkyl group R2 to the adjacent carbon atom of formula (I), or
- n = 1 and R1 and R2 together with the carbon atom located in between form a cyclohexyl ring as defined above, R3 and R5 are hydrogen and R4 is selected from the group consisting of methyl, ethyl and isopropyl and tert-butyl, or
- n = 0 and R1 and R2 together with the carbon atom located in between form a phenyl ring as defined above, R3 and R5 are hydrogen and R4 is selected from the group consisting of methyl, ethyl and isopropyl and tert-butyl, or
- n = 1, R1 is methyl and R2 is an unbranched saturated alkyl group with 4 to 6 carbon atoms.

4. Use according to claim 1 or 2, **characterized in that** the compound of formula (I) used or one, more or all compounds of formula (I) in the mixture used are selected from the group consisting of compounds, in which
- n = 1, R1 is hydrogen and R2 is a branched saturated alkyl group 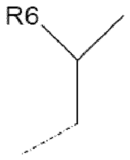 with 4 to 9 carbon atoms in total, in which R6 is a branched or unbranched saturated alkyl group with 1 to 6 carbon atoms and the dotted line represents the bond, which joins the branched saturated alkyl group R2 to the adjacent carbon atom of formula (I), or
- n = 1, R1 is methyl and R2 is an unbranched saturated alkyl group with 4 to 6 carbon atoms.

5. Use according to one of claims 1 to 3, **characterized in that** the compound of formula (I) used or one, more or all compounds of formula (I) in the mixture used are selected from the group consisting of
- 2-(3-methylbutoxy)acetaldehyde (formula (la)) (2-isopentyloxyacetaldehyde) 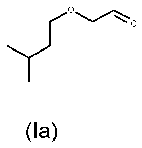
- 2-(4-isopropylcyclohexyloxy)acetaldehyde (formula (Ib)) 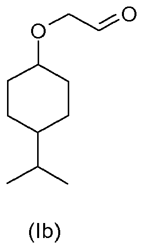
- 2-(4-isopropylphenoxy)acetaldehyde (formula (Ic)) 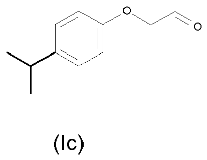
- 2-(3,5,5-trimethylhexoxy)acetaldehyde (formula (Id)) 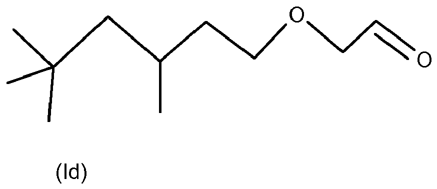
- 2-[(1-methylheptyl)oxy]acetaldehyde (formula (Ie))

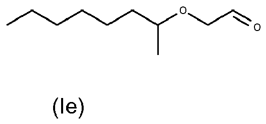

6. Compound selected from the group consisting of:
2-((4-isopropylcyclohexyloxy)acetaldehyde (formula (Ib)) 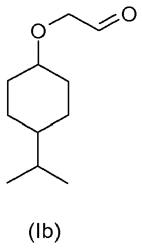 and
2-((3,5,5-trimethylhexoxy)acetaldehyde (formula (Id))

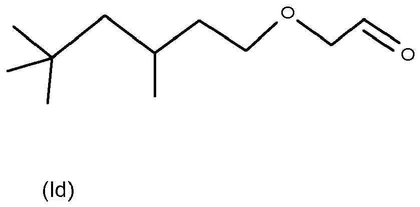

7. 1-(2,2-diethoxyethoxy)-4-isopropylcyclohexane (formula (IIb)

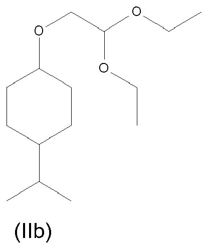

8. Mixture comprising one or more compounds of formula (I) as defined in one of the preceding claims, preferably one or more compounds of formula (I) as defined in one of claims 3 to 5
wherein the mixture
- comprises one, 2, 3, 4, 5, 6, 7, 8, 9, 10 or more further fragrance(s), with are not compounds of formula (I),
and/or
- comprises one or more solvents, which are selected from the group consisting of trialkyl citrates (preferably triethyl citrate, trimethyl citrate, tripropyl citrate, triisopropyl citrate, tributyl citrate, triisobutyl citrate or tri-sec-butyl citrate), dialkyl phthalates (preferably dimethyl phthalate, diethyl phthalate or dibutyl phthalate), benzyl benzoate, isopropyl myristate and dioctyl adipate,
and/or
- is a product selected from the group consisting of laundry and cleaning agents, hygiene or care products, in particular from the field of body and hair care, of cosmetics and housekeeping, fine fragrances (perfumes), perfume oils, air care products (in particular candles), air fresheners, means for cleaning, treating and/or tending surfaces and textile fibers.

9. Mixture according to claim 8, **characterized in that** the mixture comprises one, 2, 3, 4, 5, 6, 7, 8, 9, 10 or more further fragrances, which are not compounds of formula (I), and the total amount of the compounds of formula (I) as defined in one of the preceding claims is in the range from 0.0001 to 40 wt.-%, preferably in the range from 0.001 to 25 wt.-%, in each case with respect to the total weight of the mixture.

10. Mixture according to claim 8, **characterized in that** the mixture comprises one or more solvents, which are selected from the group of trialkyl citrates, and the total amount of compounds of formula (I) as defined in one of the preceding claims is in the range from 0.0001 to 40 wt.-%, preferably in the range from 0.001 to 25 wt.-%, in each case with respect to the total weight of the mixture.

11. Mixture according to claim 8 or 10, **characterized in that** the or one of the solvent(s) is selected from the group consisting of triethyl citrate, diethyl phthalate, benzyl benzoate, isopropyl myristate and dioctyl adipate.

12. Mixture according to claim 8, **characterized in that** the mixture is a product selected from the group consisting of laundry and cleaning agents, hygiene or care products, in particular from the field of body and hair care, of cosmetics and housekeeping, fine fragrances (perfumes), air care products (in particular candles), air fresheners, means for cleaning, treating and/or tending surfaces and textile fibers and the total amount of compounds of formula (I) as defined in one of the preceding claims is in the range from 0.0001 to 5 wt.-%, preferably in the range from 0.001 to 2.5 wt.-%, in each case with respect to the total weight of the product.

13. Method for conveying and/or enhancing
(i) a lily of the valley fragrance note
as well as (ii) optionally
(ii)a) one, more or all of the fragrance notes selected from the group consisting of flowery, fruity, watery, green and aldehyde
and/or
(ii)b) one, more or all of the impressions selected from the group consisting of natural, fresh, nurturing, gentle, warm, complex and radiant;
comprising the following step:
mixing or contacting
- one or more compounds of formula (I) as defined in one of the preceding claims, preferably one or more compounds of formula (I) as defined in one of the claims 3 to 5, or
- a mixture according to one of claims 8 to 12
in a sensorially effective amount with a product.

14. Method according to claim 13 for furnishing skin, hair, surfaces or textile fibers with
(i) a lily oft he valley fragrance note
as well as (ii) optionally
(ii)a) one, more or all of the fragrance notes selected from the group consisting of flowery, fruity, watery, green and aldehyde
and/or
(ii)b) one, more or all of the impressions selected from the group consisting of natural, fresh, nurturing, gentle, warm, complex and radiant;
comprising the following steps:
- providing
- one or more compounds of formula (I) as defined in one of the preceding claims, preferably one or more compounds of formula (I) as defined in one of claims 3 to 5, or
- a mixture according to one of claims 8 to 12,
- applying the compound(s) of formula (I) or the mixture or the perfumed product, respectively, to skin, hair, surfaces or textile fibers.

## Revendications

1. Utilisation d'un composé de la formule (I) ou d'un mélange contenant un ou plusieurs composés de la formule (I) ou d'un mélange se composant de deux ou de plusieurs composés de la formule (I), 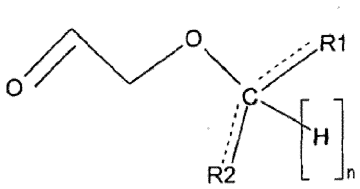 dans laquelle vaut :
n est 0 ou 1, où
(i) si n = 1
les deux paires d'une ligne continue et d'une ligne en traits interrompus dans la formule (I) représentent chacune des liaisons simples
et
a) R1 et R2 représentent chacun un groupe alkyle saturé non-ramifié, le nombre total des atomes de carbone dans les groupes R1 et R2 étant compris entre 4 et 9
ou
b) R1 et R2 forment conjointement avec l'atome de carbone disposé entre eux un cycle cyclohexyle 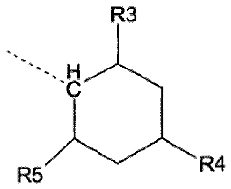 où la ligne en traits interrompus représente la liaison qui lie le cycle cyclohexyle à l'atome d'oxygène voisin dans la formule (I), et
où R3, R4 et R5 sont choisis, indépendamment les uns des autres, dans le groupe constitué par l'hydrogène, le méthyle, l'éthyle, l'isopropyle et le tert-butyle, et le nombre total des atomes de carbone du cycle cyclohexyle et des groupes R3, R4 et R5 est compris entre 6 et 10
ou
c) R1 est de l'hydrogène et R2
c1) est un groupe alkyle saturé ramifié ayant 4 à 9 atomes de carbone
ou
c2) est un cycle cyclohexyle 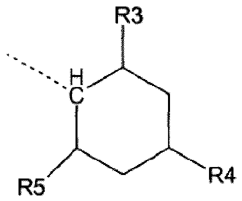 où la ligne en traits interrompus représente la liaison qui lie le cycle cyclohexyle à l'atome de carbone voisin dans la formule (I), et
où R3, R4 et R5 sont choisis, indépendamment les uns des autres, dans le groupe constitué par l'hydrogène, le méthyle, l'éthyle et l'isopropyle, et le nombre total des atomes de carbone du cycle cyclohexyle et des groupes R3, R4 et R5 est compris entre 6 et 9
ou
c3) est un cycle phényle 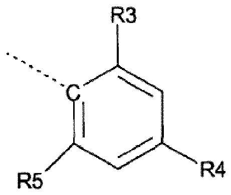
où la ligne en traits interrompus représente la liaison qui lie le cycle phényle à l'atome de carbone voisin dans la formule (I), et
où R3, R4 et R5 sont choisis, indépendamment les uns des autres, dans le groupe constitué par l'hydrogène, le méthyle, l'éthyle et l'isopropyle, et le nombre total des atomes de carbone du cycle phényle et des groupes R3, R4 et R5 est compris entre 6 et 9
et
(ii) si n = 0
les deux paires d'une ligne continue et d'une ligne en traits interrompus dans la formule (I) représentent chacune des liaisons aromatiques
et
(d) R1 et R2 forment conjointement avec l'atome de carbone disposé entre eux un cycle phényle 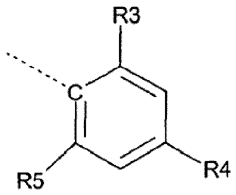
où la ligne en traits interrompus représente la liaison qui lie le cycle phényle à l'atome d'oxygène voisin dans la formule (I), et
où R3, R4 et R5 sont choisis, indépendamment les uns des autres, dans le groupe constitué par l'hydrogène, le méthyle, l'éthyle, l'isopropyle et le tert-butyle, et le nombre total des atomes de carbone du cycle phényle et des groupes R3, R4 et R5 est compris entre 6 et 10,
ou d'un composé choisi dans le groupe constitué par
3.) le 2-(1,1-diméthylpropoxy)acétaldéhyde 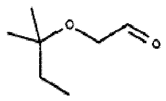
4.) le 2-(1,3-diméthylbutoxy)acétaldéhyde 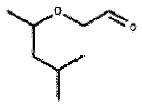
6.) le 2-[(1,5-diméthylhexyl)oxy]acétaldéhyde 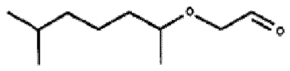
7.) le 2-(1,1-diméthylhexoxy)acétaldéhyde 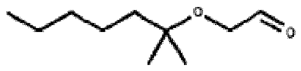
8.) le 2-[(2-éthylhexyl)oxy]acétaldéhyde 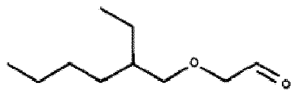
- en tant que substance odoriférante ayant une note de muguet et/ou
- comme agent destiné à augmenter la substantivité et/ou la rétention d'une préparation de substance odoriférante
et/ou
- en tant que fixateur.

2. Utilisation selon la revendication 1, dans laquelle
un ou plusieurs composés de la formule (I) tels que définis dans la revendication 1 sont utilisés pour conférer, modifier ou renforcer une note olfactive de muguet et
(i) une, plusieurs ou toutes les notes olfactives choisies dans le groupe constitué par fleurie, fruitée, aqueuse, verte et aldéhyde
et/ou
(ii) une, plusieurs ou toutes les impressions choisies dans le groupe constitué par naturelle, fraîche, de soin, douce, chaude, complexe et rayonnante.

3. Utilisation selon la revendication 1 ou 2, **caractérisée par le fait que** le composé utilisé de la formule (I) ou un, plusieurs ou tous les composés de la formule (I) dans le mélange utilisé sont choisis dans le groupe constitué par des composés dans lesquels
- n = 1, R1 est de l'hydrogène et R2 est un groupe alkyle saturé ramifié 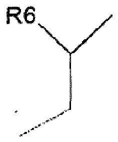 avec, dans l'ensemble, 4 à 9 atomes de carbone, où R6 est un groupe alkyle saturé ramifié ou non-ramifié ayant 1 à 6 atomes de carbone et la ligne en traits interrompus représente la liaison qui lie le groupe alkyle saturé ramifié R2 à l'atome de carbone voisin dans la formule (I), ou
- n = 1 et R1 et R2 forment conjointement avec l'atome de carbone disposé entre eux un cycle cyclohexyle comme défini ci-dessus, R3 et R5 sont de l'hydrogène et R4 est choisi dans le groupe constitué par le méthyle, l'éthyle et l'isopropyle et le tert-butyle, ou
- n = 0 et R1 et R2 forment conjointement avec l'atome de carbone disposé entre eux un cycle phényle comme défini ci-dessus, R3 et R5 sont de l'hydrogène et R4 est choisi dans le groupe constitué par le méthyle, l'éthyle et l'isopropyle et le tert-butyle, ou
- n = 1, R1 est le méthyle et R2 est un groupe alkyle saturé non-ramifié ayant 4 à 6 atomes de carbone.

4. Utilisation selon la revendication 1 ou 2, **caractérisée par le fait que** le composé utilisé de la formule (I) ou un, plusieurs ou tous les composés de la formule (I) dans le mélange utilisé sont choisis dans le groupe constitué par des composés dans lesquels
- n = 1, R1 est de l'hydrogène et R2 est un groupe alkyle saturé ramifié 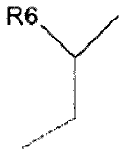 avec, dans l'ensemble, 4 à 9 atomes de carbone, où R6 est un groupe alkyle saturé ramifié ou non-ramifié ayant 1 à 6 atomes de carbone et la ligne en traits interrompus représente la liaison qui lie le groupe alkyle saturé ramifié R2 à l'atome de carbone voisin dans la formule (I), ou
- n = 1, R1 est le méthyle et R2 est un groupe alkyle saturé non-ramifié ayant 4 à 6 atomes de carbone.

5. Utilisation selon l'une quelconque des revendications 1 à 3, **caractérisée par le fait que** le composé utilisé de la formule (I) ou un, plusieurs ou tous les composés de la formule (I) dans le mélange utilisé sont choisis dans le groupe constitué par le
- 2-(3-méthylbutoxy)acétaldéhyde (formule (la)) (2-isopentyloxy acétaldéhyde) 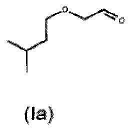
- 2-(4-isopropylcyclohexyloxy)acétaldéhyde (formule (Ib)) 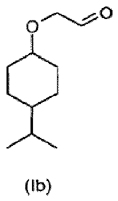
- 2-(4-isopropylphénoxy)acétaldéhyde (formule (Ic)) 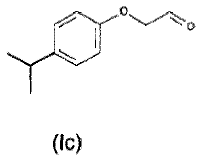
- 2-(3,5,5-triméthylhexoxy)acétaldéhyde (formule (Id)) 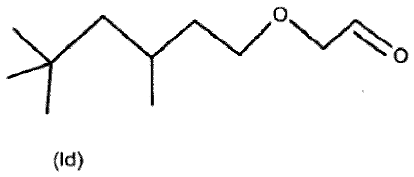
- 2-[(1-méthylheptyl)oxy]acétaldéhyde (formule (Ie))

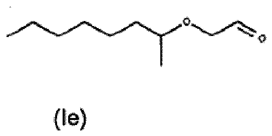

6. Composé choisi dans le groupe constitué par :
le 2-(4-isopropylcyclohexyloxy)acétaldéhyde (formule (Ib)) 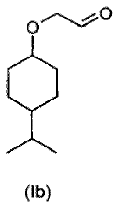 et
le 2-(3,5,5-triméthylhexoxy)acétaldéhyde (formule (Id))

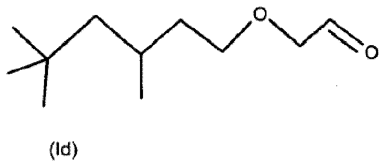

7. 1-(2,2-diéthoxyéthoxy)-4-isopropylcyclohexane (formule (IIb)

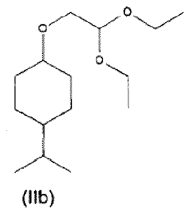

8. Mélange contenant un ou plusieurs composés de la formule (I) tels que définis dans l'une quelconque des revendications précédentes, de préférence un ou plusieurs composés de la formule (I) tels que définis dans l'une quelconque des revendications 3 à 5,
dans lequel le mélange
- contient une, 2, 3, 4, 5, 6, 7, 8, 9, 10 autres substances odoriférantes ou plus qui ne sont pas de composés de la formule (I),
et/ou
- contient un ou plusieurs solvants qui sont choisis dans le groupe constitué par les citrates de trialkyle (de préférence le citrate de triéthyle, le citrate de triméthyle, le citrate de tripropyle, le citrate de triisopropyle, le citrate de tributyle, le citrate de triisobutyle ou le citrate de tri-sec-butyle), les phtalates de dialkyle (de préférence le phtalate de diméthyle, le phtalate de diéthyle ou le phtalate de dibutyle), le benzoate de benzyle, le myristate d'isopropyle et l'adipate de dioctyle, et/ou
- est un produit choisi dans le groupe constitué par les lessives et les produits de nettoyage, les produits d'hygiène ou de soin, en particulier du domaine des soins corporels et des soins capillaires, de la cosmétique et du ménage, les parfums fins (parfums), les huiles de parfum, les produits à assainir l'air intérieur (en particulier bougies), les désodorisants, les produits de nettoyage, de traitement et/ou d'entretien de surfaces et de fibres textiles.

9. Mélange selon la revendication 8, **caractérisé par le fait que** le mélange contient une, 2, 3, 4, 5, 6, 7, 8, 9, 10 autres substances odoriférantes ou plus qui ne sont pas de composés de la formule (I), et que la quantité totale des composés de la formule (I) tels que définis dans l'une quelconque des revendications précédentes se situe dans la plage allant de 0,0001 à 40 % en poids, de préférence dans la plage allant de 0,001 à 25 % en poids, respectivement par rapport au poids total du mélange.

10. Mélange selon la revendication 8, **caractérisé par le fait que** le mélange contient un ou plusieurs solvants qui sont choisis dans le groupe des citrates de trialkyle, et que la quantité totale des composés de la formule (I) tels que définis dans l'une quelconque des revendications précédentes se situe dans la plage allant de 0,0001 à 40 % en poids, de préférence dans la plage allant de 0,001 à 25 % en poids, respectivement par rapport au poids total du mélange.

11. Mélange selon la revendication 8 ou 10, **caractérisé par le fait que** le solvant ou l'un des solvants est choisi dans le groupe constitué par le citrate de triéthyle, le phtalate de diéthyle, le benzoate de benzyle, le myristate d'isopropyle et l'adipate de dioctyle.

12. Mélange selon la revendication 8, **caractérisé par le fait que** le mélange est un produit choisi dans le groupe constitué par les lessives et les produits de nettoyage, les produits d'hygiène ou de soin, en particulier du domaine des soins corporels et des soins capillaires, de la cosmétique et du ménage, les parfums fins (parfums), les produits à assainir l'air intérieur (en particulier bougies), les désodorisants, les produits de nettoyage, de traitement et/ou d'entretien de surfaces et de fibres textiles, et que la quantité totale des composés de la formule (I) tels que définis dans l'une quelconque des revendications précédentes se situe dans la plage allant de 0,0001 à 5 % en poids, de préférence dans la plage allant de 0,001 à 2,5 % en poids, respectivement par rapport au poids total du produit.

13. Procédé destiné à conférer et/ou à renforcer
(i) une note olfactive de muguet
ainsi que, le cas échéant, (ii)
(ii)a) une, plusieurs ou toutes les notes olfactives choisies dans le groupe constitué par fleurie, fruitée, aqueuse, verte et aldéhyde
et/ou
(ii)b) une, plusieurs ou toutes les impressions choisies dans le groupe constitué par naturelle, fraîche, de soin, douce, chaude, complexe et rayonnante ;
comprenant l'étape suivante :
mélanger ou mettre en contact
- un ou plusieurs composés de la formule (I) tels que définis dans l'une quelconque des revendications précédentes, de préférence un ou plusieurs composés de la formule (I) tels que définis dans l'une quelconque des revendications 3 à 5, ou
- un mélange selon l'une quelconque des revendications 8 à 12 en une quantité sensoriellement efficace avec un produit.

14. Procédé selon la revendication 13, destiné à pourvoir la peau, les cheveux, des surfaces ou fibres textiles
(i) d'une note olfactive de muguet
ainsi que, le cas échéant, (ii)
(ii)a) d'une, de plusieurs ou de toutes les notes olfactives choisies dans le groupe constitué par fleurie, fruitée, aqueuse, verte et aldéhyde
et/ou
(ii)b) d'une, de plusieurs ou de toutes les impressions choisies dans le groupe constitué par naturelle, fraîche, de soin, douce, chaude, complexe et rayonnante ;
comprenant les étapes suivantes :
- fournir
- un ou plusieurs composés de la formule (I) tels que définis dans l'une quelconque des revendications précédentes, de préférence un ou plusieurs composés de la formule (I) tels que définis dans l'une quelconque des revendications 3 à 5, ou
- un mélange selon l'une quelconque des revendications 8 à 12,
- appliquer le(s) composé(s) de la formule (I) ou bien le mélange ou le produit parfumé sur la peau, les cheveux, les surfaces ou les fibres textiles.
